# EUROPEAN PATENT SPECIFICATION

(11) **EP 3 454 910 B1**
(45) Date of publication and mention of the grant of the patent: **05.11.2025**
(21) Application number: 17723742.7
(22) Date of filing: 03.05.2017
(51) Int. Cl.: A61K 48/00, C07K 14/47

(54) **TREATMENT OF COMPLEMENT-MEDIATED DISORDERS**
BEHANDLUNG VON KOMPLEMENTVERMITTELTEN KRANKHEITEN
TRAITEMENT DE TROUBLES MÉDIÉS PAR LE COMPLÉMENT

(30) Priority: 09.05.2016 GB 201608046
(43) Date of publication of application: 20.03.2019
(73) Proprietor: Cambridge Enterprise Limited, Trinity Lane, Cambridge CB2 1TN (GB); The Sydney Children's Hospitals Network (Randwick and Westmead), Westmead, NSW 2145 (AU)
(72) Inventor: LACHMANN, Peter, Cambridge Cambridgeshire CB3 0ES (GB); ALEXANDER, Ian, Sydney, New South Wales 2145 (AU)
(74) Representative: D Young & Co LLP
(86) International application number: PCT/GB2017/051233
(87) International publication number: WO 2017/194912

(56) References cited:
- WO-A2-2010/103291
- GENECARDS HUMAN GENE DATABASE: "CFI Gene - GeneCards | CFAI Protein | CFAI Antibody", 23 March 2016 (2016-03-23), XP055387757, Retrieved from the Internet <URL:https://web.archive.org/web/20160323030335/http://www.genecards.org/cgi-bin/carddisp.pl?gene=CFI> [retrieved on 20170704]
- ANONYMOUS: "CFI Adenovirus/AAV from Vector BioLabs", 4 July 2017 (2017-07-04), XP055387768, Retrieved from the Internet <URL:http://www.vectorbiolabs.com/vbs/virus-search.html?search=cfi&from_page=/vbs/search.html?keyword=cfi#> [retrieved on 20170704]
- SIOBHAN M. CASHMAN ET AL: "Adenovirus-mediated delivery of Factor H attenuates complement C3 induced pathology in the murine retina: a potential gene therapy for age-related macular degeneration", JOURNAL OF GENE MEDICINE, vol. 17, no. 10-12, 1 October 2015 (2015-10-01), US, pages 229 - 243, XP055336604, ISSN: 1099-498X, DOI: 10.1002/jgm.2865
- THOMAS C E ET AL: "Rapid uncoating of vector genomes is the key to efficient liver transduction with pseudotyped adeno-associated virus vectors", JOURNAL OF VIROLOGY, THE AMERICAN SOCIETY FOR MICROBIOLOGY, US, vol. 78, no. 6, 1 March 2004 (2004-03-01), pages 3110 - 3122, XP002312778, ISSN: 0022-538X, DOI: 10.1128/JVI.78.6.3110-3122.2004
- HORIE-INOUE KUNIKO ET AL: "Genomic aspects of age-related macular degeneration", BIOCHEMICAL AND BIOPHYSICAL RESEARCH COMMUNICATIONS, ELSEVIER, AMSTERDAM, NL, vol. 452, no. 2, 8 August 2014 (2014-08-08), pages 263 - 275, XP029063982, ISSN: 0006-291X, DOI: 10.1016/J.BBRC.2014.08.013
- CELL BIOLABS: "Product Manual AAV-2 Helper Free Packaging System Catalog Number VPK-402 1 kit", 1 January 2015 (2015-01-01), XP055387937, Retrieved from the Internet <URL:https://www.cellbiolabs.com/sites/default/files/VPK-402-aav-helper-free-expression-system.pdf> [retrieved on 20170705]

## Description

This invention relates to methods of treatment of complement-mediated disorders, in particular disorders associated with over-activity of the complement C3b feedback cycle (for example, age-related macular degeneration (AMD)), using gene therapy to elevate levels of complement Factor I (or fragments or derivatives thereof), to recombinant viral vectors encoding Factor I (or fragments or derivatives thereof), to recombinant virus particles encapsidating the vectors, and to their use in the methods of treatment.

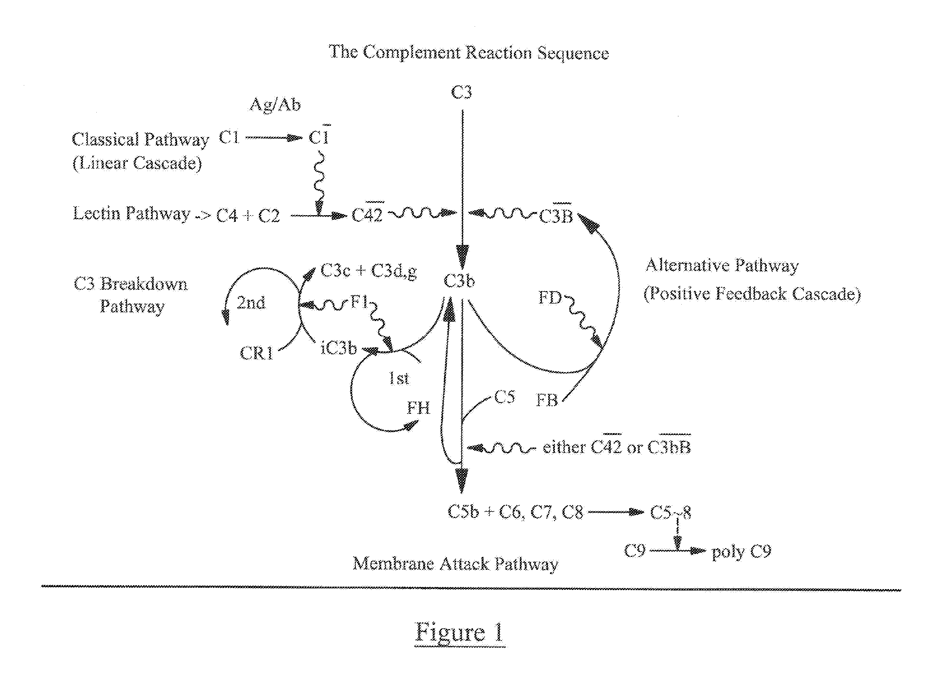

The complement system forms a first line of defence against infections by triggering inflammatory responses to alert the immune system to impending danger. It has an essential role in tagging microbes and infected or damaged cells to promote their killing by lysis or phagocytic clearance. The complement system can be activated by three different mechanisms, the classical, lectin, and alternative pathways (see Figure 1). All three pathways converge at the cleavage of the abundant plasma protein C3, which enables activation of the terminal complement steps as well as other complement effector mechanisms.

Today, it is generally accepted that the alternative pathway is activated either by one of the other two complement pathways, giving rise to positive feedback amplification, or by iC3 (or C3(H₂O)), which is the result of the spontaneous hydrolysis of the internal thioester bond in C3, or C3b which are constantly generated in serum at very low concentrations. This "tickover" provides the required minimal amounts of activated C3 that can be bound by Factor B which is further cleaved by Factor D to form the initial C3 convertase complex iC3Bb, that can convert native C3 to C3b. C3b undergoes a structural rearrangement that enables the serine protease precursor Factor B to bind to C3b. Factor D then can join the complex and cleave Factor B into Bb and Ba. C3bBb is the alternative pathway C3 convertase. Factor D does not cleave Factor B in serum unless Factor B is bound to either C3b or iC3. The newly generated C3b can bind covalently to the surface of pathogens (or any other nearby surface), where it can be bound by another molecule of Factor B. This binding is stabilized by Properdin that increases the half-life of the alternative pathway C3 convertase by antagonizing the functional activity of Factor H.

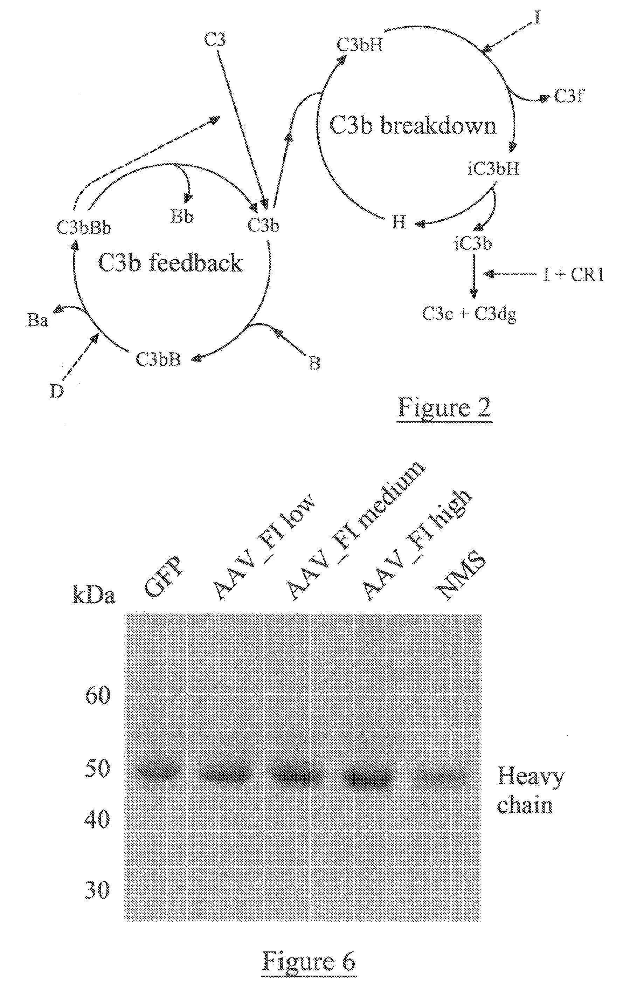

The alternative pathway is governed by a balance between two competing cycles that both act on C3b. These are the C3b feedback and C3b breakdown cycles, which lie at the heart of complement activation (see Figure 2). Once iC3 or C3b is formed and deposited on a surface, two different reactions can occur independent of how C3b was generated in the first place: it can either be bound by Factor B (feedback) or by Factor H (or membrane bound FI cofactors CD46 or CD35) (breakdown).

Binding to C3b (or C3(H₂O)) either leads to amplification of the C3 convertase (in the presence of Factor B, Factor D and Properdin) and initiation of the assembly of the membrane attack complex (MAC) or to inactivation of C3b (in the presence of Factor H and Factor I). Whether amplification or inactivation occurs depends solely on the nature of the surface to which C3b is attached. On so-called "protected surfaces", Factor H binding (and therefore also Factor I cleavage of C3b) is impaired. For example, Factor H binding to C3b on lipopolysaccharide (LPS) of *Escherichia coli 04* is far weaker than that of Factor B.

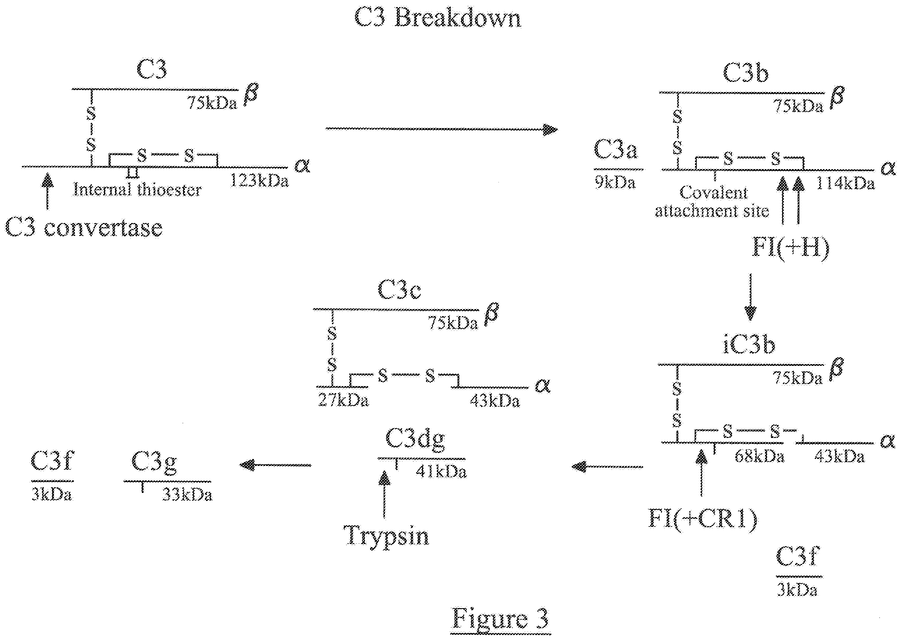

The breakdown of C3 during complement activation is depicted in Figure 3. C3 (185 kDa) consists of two chains, the α and the β chain, while in C3b, the N-terminal peptide, C3a (9kDa), is cleaved off by a C3 convertase. Cleavage of C3b is achieved by two Factor I-mediated cuts in the α'-chain of C3b. A small fragment called C3f (3kDa) is released, dividing the α-chain into a 68 and a 43kDa fragment. This proteolysed C3 is now called iC3b and it can no longer take part in a C3 or C5 convertase. It is however an important mediator of complement induced inflammation by virtue of its reactivity with the complement receptor CR3 (CD11b,CD18) on neutrophils. This first cleavage to iC3b occurs quite quickly and uses Factor H as a co-factor, while the second cleavage of iC3b is much slower. Only in the presence of the co-factor complement receptor 1 (CR1) can iC3b be further cleaved by Factor I. Factor I cleaves iC3b again in the 68 kDa fragment which releases a large part of the molecule, called C3c. C3dg remains bound to the surface by a covalent bond (either an ester bond to a sugar or an amide bond to a protein). C3dg does not react with CR3 and is not an inflammatory mediator.

Genetic variations in complement proteins that affect their function are associated with resistance to infectious diseases and susceptibility to various inflammatory diseases. The best-described polymorphisms are associated with age-related macular degeneration (AMD), dense deposit disease (DDD), and atypical haemolytic uremic syndrome (aHUS).

AMD is the most common disease affected by polymorphisms in the alternative pathway. It is associated with several common variants in Factor H and FH-related proteins: FH haplotype 1, which includes the fH_{Y402H} SNP (Haines, et al., Science, 308(5720):419-421, April 2005; Edwards, et al., Science, 308(5720):421-424, April 2005; Klein, et al., Science, 308(5720):385-389, April 2005); *FH* haplotype 2, which includes the fH_{V62I} SNP (Hageman et al., Proc. Natl. Acad. Sci. U.S.A., 102(20):7227-7232, May 2005); and *FH* haplotype 3, which includes the deletion of *CFHR1*/*CFHR3,* protective (Hughes et al., Nat. Genet., 38(10):1173-1177, October 2006). Inheriting two copies of the FH haplotype 1 was shown by cited studies to increase the risk of AMD sevenfold. Other AMD-associated genetic variations are in Factor B and C3 (C3 R102G (Heurich, et al., Proc. Natl. Acad. Sci. U.S.A., 108(21):8761-8766, May 2011)). Quite recently, rare Factor I mutations that predispose to low Factor I serum levels have been associated with a significantly increased risk for advanced AMD (Kavanagh, et al., Hum. Mol. Genet., 24(13):3861-3870, July 2015). The polymorphisms mentioned above are listed in Table 1 below.

**Table 1. Polymorphisms associated with age-related macular degeneration**

| **SNP name** | **Coding variant** | **Minor Allele** | **Odds Ratio (OR)** |
|---|---|---|---|
| rs1061170 | fH _{Y402H} | H (risk) | 1.99-2.5^{1,2} |
| rs800292 | fH _{V62I} | I (protective) | 0.54³ |
| ΔCFHR1/CFHR3 | deletion of CFHR 1 and 3 | [Δ (protective)] | 0.48¹ |
| rs641153 | fB _{R32Q} | R (risk) | 0.32⁴ |
| rs2230199 | C3 _{R102G} (also S/F) | G (risk) | 2.6⁵ |

| | | | |
|---|---|---|---|
| ¹Hughes, *et al., supra;* ²Zareparsi, et al., Am. J. Hum. Genet., 77(1):149-153, July 2005; ³Hageman, *et al., supra;* ⁴Gold, et al., Nat. Genet., 38(4):458-462, April 2006; ⁵Yates, et al., New England Journal of Medicine, 357(6):553-561, August 2007. | | | |

Dense deposit disease, DDD, is clinically linked to AMD in that patients with DDD also develop retinal changes as in AMD, but at a much earlier age (if they survive) than "AMD-only" patients. In fact, polymorphisms in H1 and H2 haplotypes also confer an increased or decreased risk for DDD, respectively, as reported for AMD (Pickering, et al., J. Exp. Med., 204(6):1249-1256, June 2007) as well as polymorphisms in C3. Combinations of several risk haplotypes increase DDD risk dramatically (Abrera-Abeleda, et al., J. Am. Soc. Nephrol., 22(8):1551-1559, August 2011). It is known from mouse studies that the development of DDD is entirely dependent on the capacity to generate iC3b (Rose, et al., J. Clin. Invest., 118(2):608-618, February 2008). There is an absolute need in disease development for the presence of Factor I. Complete Factor I deficiency abolishes risk for DDD and AMD because of the absence of the inflammatory mediator iC3b.

Both, AMD and DDD are associated with changes in fluid phase alternative pathway regulation and can therefore be seen as systemic, rather than eye- or kidney-specific diseases. The reason why these two organs are particularly affected probably lies in the exact distribution of complement regulator proteins and organ architecture. In both organs, there is a separation of erythrocytes and plasma and a basement membrane that is in direct contact with plasma, making protection from complement attack much more dependent on Factor H co-factor and Factor I activity. Most polymorphisms predisposing to AMD and DDD are found in regions of Factor H that are responsible for C3 binding.

Atypical haemolytic uremic syndrome (aHUS) is also associated with mutations in alternative pathway genes, particularly in the factor H gene (half of all aHUS cases), but as opposed to AMD and DDD, these mutations were usually found in the C-terminus of Factor H, a region responsible for sequestering Factor H to surfaces (Manuelian, et al., J. Clin. Invest., 111(8):1181-1190, April 2003). Other predisposing polymorphisms were found in the C3 or CFB gene (Goicoechea de Jorge, et al., Proc. Natl. Acad. Sci. U.S.A., 104(1):240-245, January 2007; Frémeaux-Bacchi, et al., Blood, 112(13):4948-4952, December 2008). In summary, risk of aHUS is highly increased in the presence of risk polymorphisms that promote inappropriate alternative pathway activation on cell surfaces (Rodriguez de Córdoba, et al., Biochimica et Biophysica Acta (BBA) - Molecular Basis of Disease, 1812(1):12-22, January 2011).

Genome-wide association studies have identified further polymorphisms that lie in, for example the *CR1* gene, clusterin (both further predisposing to Alzheimer's disease (AD) in individuals that already have one or two copies of the AD predisposing APOE-ε4 allele) (Harold, et al., Nat. Genet., 41(10):1088-1093, October 2009; Lambert, et al., Nat. Genet., 41(10):1094-1099, October 2009), and C5 (associated with rheumatoid arthritis) (Kurreeman, et al., PLoS Med., 4(9):e278, September 2007). It should also be noted that amplification of complement and its effects via the feedback cycle is inherent in all complement pathways, so its manipulation has the potential to improve other pathophysiological processes as well.

Most of the polymorphisms mentioned above have in common that the disease allele predisposes to an increase in the alternative pathway feedback activity that amplifies all complement pathways irrespective of triggering stimulus (Lachmann, Adv. Immunol., 104:115-149, 2009). A hyperinflammatory complotype (defined as representing the pattern of genetic variants in complement genes inherited by an individual which alters risk for both inflammatory disorders and infectious diseases involving complement) thus protects against infection, particularly in early childhood, but comes at the expense that, in later life when an individual already has formed IgG antibodies against commonly invading pathogens, it predisposes to inflammatory diseases. Since, the list of diseases affected by complotype grows steadily as genetic studies implicate known or novel complement polymorphisms (Harris, et al., Trends Immunol., 33(10):513-521, October 2012), there is considerable interest and need in finding methods for reversing this hyperactivity of the C3b feedback cycle.

The balance of the feedback loop, which determines the amount of generated C3b, can be tipped at both ends. More C3b can be generated either by amplification of the feedback loop or by inhibition of the C3b breakdown mechanism. The feedback loop can be amplified by increase of C3b input (for example, by more C3 convertases from the classical/lectin pathways), by acceleration of the feedback reactions (for example, by gain of function mutants of Factor B, or an increase of Factor D or magnesium ions), by addition of cobra venom factor (which has C3b-like properties and can bind Factor B and form a C3 convertase after cleavage of Factor B by Factor D), or by stabilisation of the alternative pathway C3 convertase (for example, by Properdin or nephritic factors). C3b breakdown can be prevented by either absence or low concentrations of Factor H and Factor I or membrane cofactor protein (MCP) or by a susceptible C3 genotype (for example, in C3F, SNP: R102G has a lower affinity to Factor H than C3S and is therefore more slowly cleaved by Factor I). C3 breakdown is also impeded on protected surfaces. Negative regulation of the C3b feedback cycle is achieved by promotion of C3b breakdown. This is done either by raising the concentration of Factor H or Factor I, or by inhibition of Factor D, Factor B, or Properdin. Particular targets for downregulation of the C3b feedback cycle are Factor D, Factor H and Factor I.

A therapy for the treatment of geographic atrophy, a late form of AMD, has already been developed and is currently in a phase III clinical study (MAHALO study by Genentech/Roche). Here, a humanised monoclonal inhibitory antibody to Factor D (lampalizumab) administered by intravitreal injection is used to stop the rate of progression of geographic atrophy. Factor D is present in very low serum concentrations and is an essential factor for the alternative pathway. Nevertheless, due to its small size (27kDa), Factor D is rapidly cleared out by the kidneys and quickly re-synthesized. Thus, the anti-Factor D treatment works for local inhibition of Factor D in a small compartment such as the retina, but not systemically. The route of administration, intravitreal injection, is not without associated risk, which is another drawback of this treatment.

Regarding the other two target proteins, it has been known since the seventies that the C3b feedback cycle can be down-regulated by increasing the plasma concentrations of Factor H and I (Nydegger, et al., J Immunol, 120(4):1404-1408, January 1978; Lachmann and Halbwachs, Clin. Exp. Immunol., 21(1):109-114, July 1975). New arising genomic data of steadily increasing associations of complement and/or alternative pathway disorders with a number of diseases (for example, Harris *et al.,* 2012 *(supra);* Haines, *et al.,* 2005 *(supra);* Hageman, *et al.,* 2005 *(supra);* Kavanagh, et al., Hum. Mol. Genet., 24(13):3861-3870, July 2015) supports the use of Factor I or Factor H in therapy against uncontrolled pathway associated disorder. Nevertheless, Factor I is a better target for several reasons. First of all, it is present in much lower concentrations than Factor H (= 35µg/ml (FI) and ≈ 200-500µg/ml (FH), respectively). Lower quantities are easier to handle and also reduce the cost of therapy. Further, there are variants of Factor H, called Factor H-related proteins 1-5 (FHR1-5), that compete with Factor H for binding to surfaces. FHR1, 2 and 5 contain a shared dimerization motif that enables formation of three homodimers and three heterodimers which have significantly increased avidity and out-compete Factor H at physiologically relevant concentrations. Increasing Factor H concentration probably does not overcome the dominant pathogenic effects of these competitive antagonists. The last and most important reason is that Factor I is the only regulator that not only promotes cleavage of C3b to iC3b but also accelerates the breakdown of iC3b. Under physiological conditions, the affinity of Factor H to iC3b is too low to form a complex. Since iC3b reacting with the complement receptor CR3 is a major mechanism by which complement activation gives rise to inflammation, the breakdown of iC3b to C3dg is essential for reducing complement-induced inflammation (Lachmann, Adv. Immunol., 104:115-149, 2009).

Studies of AMD are hindered by the lack of optimal animal models that replicate the human disease, in particular because of the absence of a macula in mice. However, we have recognized that AMD has an underlying systemic, rather than an eye-specific defect, which is shown by the increasing list of AMD-associated polymorphisms in complement proteins and regulators. We have appreciated that the key to a therapy for AMD lies in the control of systemic alternative pathway regulation. Down-regulation of the alternative pathway C3b feedback cycle may be achieved by increasing plasma levels of Factor I. WO 2010/103291 describes methods for treatment of AMD by supplementation of plasma-purified or recombinant Factor I.

The effects of Factor I supplementation (in the presence of zymosan as alternative pathway activator) on iC3b formation, and its subsequent breakdown to C3dg has been measured in three different complotypes (Lay, et al., Clin. Exp. Immunol., 181(2):314-322, August 2015; Lachmann, et al., Clin. Exp. Immunol., September 2015). Chosen complotypes were divided into susceptible- or protective-homozygous and heterozygous with respect to three common polymorphisms associated with AMD, C3 S/F_{R102G}, fH_{Y402H}, and fH_{V62I}. The results show not only that the susceptible complotypes have delayed and much less iC3b to C3dg conversion than both the protected and heterozygous group, but also that by increasing the total Factor I concentration, the susceptible genotype can be "converted" to a protected one and can overcome disadvantageous polymorphisms in *CFH* and *C3*. Supplementation with 22µg/ml Factor I (less than normal FI concentration) converts the "at risk" genotype to a protected genotype (Lachmann *et al.,* 2015 (*supra*))*.* The presence of just three loci therefore has striking influence on the regulation of alternative pathway complement activation by Factor I in that the hyperinflammatory complotype is more resistant to down-regulation by increased Factor I concentration. By increasing plasma concentrations of Factor I the effects of a disadvantageous complotype may be reversed, and disease progression slowed down.

Gene therapy is the delivery of genetic material into a patient with therapeutic intent. In the case of *in vivo* gene therapy, a vector is injected into the patient and enters target cells where a transgene carried by the vector is subsequently expressed. Initially, gene therapy focused on orphan disease with monogenetic defects that require a straightforward but comparably easy approach, e.g. severe combined immunodeficiency (SCID). Early successes allowed the field to expand to acquired diseases such as cancer, cardiovascular diseases, neurodegenerative disorders, and infectious diseases. Notable successes have been achieved in the treatment of hemophilia B and lipoprotein lipase deficiency. However, no gene therapy product has yet been approved by the Food and Drug Administration (FDA), despite numerous clinical trials. In Europe, the first gene therapy, Glybera, was approved by the European Medicines Agency in 2012. Glybera is a treatment for a rare monogenetic disease, lipoprotein lipase deficiency (LPLD), and consists of the missing gene packaged into a recombinant viral vector that is injected intramuscularly.

Current gene therapy treatments rely on gene replacement to replace a defective gene, rather than to achieve over-expression of an existing, functional gene. We have found, surprisingly, that plasma levels of Factor I can be significantly increased in mice by gene therapy using a recombinant viral vector.

The present invention is based on the recognition that negative regulation of the complement C3b feedback cycle can be achieved by *in vivo* over-expression in plasma, by gene therapy, of Factor I. The resulting re-balancing of the feedback loop of the alternative pathway will promote C3b and iC3b breakdown and thus remove major disease factors in complement-mediated disorders, particularly disorders that have an underlying defect in alternative pathway regulation.

The references to the methods of treatment by therapy or surgery or in vivo diagnosis methods in the description and examples of this description are to be interpreted as references to compounds, pharmaceutical compositions and medicaments of the present invention for use in those methods.

WO 2010/103291 A2 discloses that raising the level of Factor I above physiological levels can be used to treat diseases in which the underlying pathology is linked to overactivity of the C3b-feedback cycle and the generation and pro-inflammatory effects of iC3b.

According to the invention there is provided a recombinant adeno-associated virus (rAAV) particle for use in preventing, treating, or ameliorating a complement-mediated disorder in a subject in need thereof, wherein the rAAV particle encapsidates a rAAV vector which comprises a nucleic acid encoding Factor I, or a fragment or derivative thereof that retains C3b-inactivating and iC3b-degradation activity, such that a therapeutically effective amount of the encoded Factor I, or the fragment or derivative thereof, is expressed from the nucleic acid in the subject, thereby increasing the level of C3b-inactivating and iC3b-degradation activity in the subject, wherein the rAAV particle is administered intravenously to the subject,
wherein the encoded Factor I, or the fragment or derivative thereof, is expressed from a liver specific promoter,
wherein the rAAV particle is pseudotyped to confer liver tropism,
wherein the derivative of Factor I is a polypeptide that retains C3b-inactivating and iC3b-degradation activity and has at least 70% amino acid sequence identity across its entire length to human Factor I with an amino acid sequence of SEQ ID NO: 2 or 4, and
wherein the complement-mediated disorder is age-related macular degeneration (AMD), dense deposit disease (DDD), atypical haemolytic uraemic syndrome (aHUS), C3 glomerulopathies, membranoproliferative glomerulonephritis Type 2 (MPGN2), atherosclerosis, chronic cardiovascular disease, Alzheimer's disease, systemic vasculitis, paroxysmal nocturnal haemoglobinuria (PNH), inflammatory or autoinflammatory diseases of old age, membranoproliferative glomerulonephritis Type 1 (MPGN1), membranoproliferative glomerulonephritis Type III (MPGN3), Guillain-Barré syndrome, Henoch-Schonlein purpura, IgA nephropathy, or membranous glomerulonephritis.

The invention may be used to effect a systemic increase in the level of C3b-inactivating and iC3b-degradation activity in a subject.

In particular embodiments, the level of C3b-inactivating and iC3b-degradation activity in the subject is increased to a level that exceeds a normal level.

There is also provided according to the invention a recombinant adeno-associated virus (rAAV) vector, which comprises a nucleic acid encoding Factor I, or a fragment or derivative thereof that retains C3b-inactivating and iC3b-degradation activiy, wherein the nucleic acid encoding Factor I, or the fragment or derivative thereof, is operably linked to a promoter, wherein the promoter is a liver-specific promoter, and wherein the derivative of Factor I is a polypeptide that retains C3b-inactivating and iC3b-degradation activity and has at least 70% amino acid sequence identity across its entire length to human Factor I with an amino acid sequence of SEQ ID NO: 2 or 4.

There is further provided according to the invention a recombinant adeno-associated virus (rAAV) virus particle, which comprises a rAAV capsid encapsidating a rAAV vector of the invention, wherein the rAAV particle is pseudotyped to confer liver tropism.

There is further provided according to the invention a pharmaceutical composition, which comprises: a rAAV vector according to the invention, or a rAAV particle according to the invention, and a pharmaceutically acceptable carrier, excipient, or diluent, preferably wherein the pharmaceutical composition is suitable for intravenous administration.

There is further provided according to the invention a rAAV vector according to the invention, or a rAAV particle according to the invention or a pharmaceutical composition according to the invention for use as a medicament.

The viral genome is comprised of genes and cis-acting regulatory sequences which are spatially separated in most viruses. This arrangement is used to design viral vectors. The viral genes which are responsible for replication or caspid/envelope proteins are exchanged with therapeutic transgenes that are then flanked on both ends by the regulatory *cis*-acting sequences (Thomas, et al., Nat. Rev. Genet., 4(5):346-358, May 2003). The deleted genes work in *trans* and can be provided either by a packaging cell line that has the viral genes incorporated into the genome, or by heterologous plasmids that are co-transfected with the viral vector(Kay et al., Nat. Med., 7(1):33-40, January 2001). In this way, replication deficient virus particles harbouring the transgene can be produced that are able to transduce their target cell.

A "recombinant viral vector" refers to a recombinant polynucleotide vector comprising one or more heterologous sequences, i.e. nucleic acid sequence not of viral origin. A recombinant viral vector can be in any of a number of forms, including plasmids, linear artificial chromosomes, complexed with lipids, encapsulated within liposomes, and encapsidated in a virus particle. A recombinant viral vector can be packaged into a virus capsid to generate a recombinant virus particle. A "recombinant virus particle" refers to a virus particle composed of at least one virus capsid protein and an encapsidated recombinant viral vector genome.

Recombinant viral vectors include recombinant viral vectors derived from retroviruses, lentiviruses, herpes simplex-1 viruses (HSV-1), adenoviruses, and adeno-associated viruses (AAVs) (Thomas *et al., supra -* see Table 1 of this document for a comparison of the relative advantages and disadvantages of use of these vectors in gene therapy).

Viral vectors can be divided into integrating and non-integrating vectors. Integrating vectors, such as retroviruses and lentiviruses, insert the transgene permanently into the host cellular chromosome. Non-integrating vectors, such as adenovirus and HSV-based vectors, mediate transgene expression from episomes. AAV-based vectors are predominantly non-integrating vectors (<10% integrated). Compared to integrating vectors, which will be inherited to every daughter cell, non-integrating vectors will be quickly diluted out in rapidly dividing cells but also do not pose the risk of insertional mutagenesis. Therefore, retroviruses are usually used for transfection of cells which undergo rapid cell divisions and differentiation (for example, haematopoietic stem cells), and non-integrating viruses are used for post-mitotic tissue (for example, the liver, muscle or eye).

In particular embodiments of the invention, the recombinant viral vector is a non-integrating (or predominantly non-integrating, i.e. less than 50%, for example less than 40%, 30%, 20%, or 10%, of persistent vector genomes being integrated *in vivo*), episomal viral vector.

In particular embodiments of the invention, the recombinant virus particle infects non-dividing cells, for example liver cells (hepatocytes).

In other embodiments of the invention, the recombinant virus particle infects dividing cells, for example B-lymphocytes.

In particular embodiments, the recombinant virus particle infects cells that result in expression and secretion of the encoded Factor I, or fragment or derivative thereof, into the subject's bloodstream. Suitable cell types include liver cells (hepatocytes), and B-lymphocytes.

The recombinant virus particle should be capable of transducing cells, which are the intended target of expression of the encoded Factor I or fragment or derivative. In particular embodiments, the recombinant virus particle is capable of transducing liver cells (in particular hepatocytes).

In some embodiments, the recombinant viral vector comprises nucleic acid encoding the Factor I, or fragment or derivative thereof, flanked by viral cis-acting regulatory sequences, such as inverted terminal repeats (ITRs).

Exemplary promoters include, but are not limited to, the cytomegalovirus (CMV) immediate early promoter, the RSV LTR, the MoMLV LTR, the phosphoglycerate kinase- 1 (PGK) promoter, a simian virus 40 (SV40) promoter and a CK6 promoter, a transthyretin promoter (TTR), a TK promoter, a tetracycline responsive promoter (TRE), an HBV promoter, a human alpha-1-anti-trypsin (hAAT) promoter, an albumin promoter, a liver-specific promoter (LSP), chimeric
liver-specific promoters (LSPs), the E2F promoter, the telomerase (hTERT) promoter; the cytomegalovirus enhancer/chicken beta-actin/Rabbit β-globin promoter (CAG promoter; Niwa et al., Gene, 1991, 108(2): 193-9) and the elongation factor 1-alpha promoter (EFI-alpha) promoter (Kim et al., Gene, 1990, 91(2):217-23 and Guo et al., Gene Ther., 1996, 3(9):802-10). Other examples include a human β-glucuronidase promoter, a β-actin promoter, a chicken β-actin (CBA) promoter, a cytomegalovirus enhancer linked to a CBA promoter, a retroviral Rous sarcoma virus (RSV) LTR promoter, or a dihydrofolate reductase promoter.

In the present invention, the promoter promotes expression of the nucleic acid encoding Factor I, or the fragment or derivative thereof, in cells of the liver, for example, in hepatocytes.

Examples of liver-specific promoters suitable for use in the present invention include HLP, LP1, HLP2, hAAT, HCR-hAAT, ApoE-hAAT, and LSP.

These liver-specific promoters are described in more detail in the following references:
HLP: Mcintosh J. et al, Blood 2013 Apr 25, 121(17):3335-44, and WO 2011/005968;
LP1: Nathwani et al, Blood 2006 April 1, 107(7): 2653-2661, and WO06/036502;
HCR-hAAT: Miao et al, Mol Ther. 2000;1: 522-532;
ApoE-hAAT: Okuyama et al, Human Gene Therapy, 7, 637-645 (1996);
LSP: Wang et al, Proc Natl Acad Sci USA 1999 March 30, 96(7): 3906-3910; and
HLP2: WO 2016/075473.

The promoter can be a constitutive, inducible or repressible promoter. Exemplary promoters and descriptions may be found, e.g., in US 201410335054.

Examples of constitutive promoters include, without limitation, the retroviral Rous sarcoma virus (RSV) LTR promoter (optionally with the RSV enhancer), the cytomegalovirus (CMV) promoter (optionally with the CMV enhancer) (see, e.g., Boshart et al., Cell, 41:521-530 (1985)), the SV40 promoter, the dihydrofolate reductase promoter, the 13-actin promoter, the phosphoglycerol kinase (PGK) promoter, and the EF1a promoter (invitrogen).

Inducible promoters allow regulation of gene expression and can be regulated by exogenously supplied compounds, environmental factors such as temperature, or the presence of a specific physiological state, e.g., acute phase, a particular differentiation state of the cell, or in replicating cells only. Inducible promoters and inducible systems are available from a variety of commercial sources, including, without limitation, Invitrogen, Clontech and Ariad. Many other systems have been described and can be readily selected by one of skill in the art. Examples of inducible promoters regulated by exogenously supplied promoters include the zinc-inducible sheep metallothionine (MT) promoter, the dexamethasone (Dex)-inducible mouse mammary tumor virus (MMTV) promoter, the T7 polymerase promoter system (WO 98/10088); the ecdysone insect promoter (No et al., Proc. Natl. Acad. Sci. USA, 93:3346-3351 (1996)), the tetracycline-repressible system (Gossen et al., Proc. Natl. Acad. Sci. USA, 89:5547-5551 (1992)), the tetracycline-inducible system (Gossen et al., Science, 268:1766-1769 (1995), see also Harvey et al., Curr. Opin. Chem. Biol., 2:512-518 (1998)), the RU486-inducible system (Wang et al., Nat. Biotech., 15:239-243 (1997) and Wang et al., Gene Ther., 4:432-441 (1997)) and the rapamycin-inducible system (Magari et al., J. Clin. Invest., 100:2865-2872 (1997)). Still other types of inducible promoters which may be useful in this context are those which are regulated by a specific physiological state, e.g., temperature, acute phase, a particular differentiation state of the cell, or in replicating cells only.

In another embodiment, the native promoter, or fragment thereof, for the Factor I, or fragment or derivative there, may be used. The native promoter can be used when it is desired that expression of the Factor I, or fragment or derivative thereof, should mimic the native expression (as long as, following expression, the level of C3b-inactivating and iC3b-degradation activity in the subject is increased or exceds a normal level). The native promoter may be used when expression of the transgene must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression.

The recombinant viral vector may include any regulatory sequence required to establish expression of the encoded Factor I, or the fragment or derivative thereof, in the target tissue or cell. For example, the recombinant viral vector may comprise an expression cassette comprising the nucleic acid encoding Factor I, or the fragment or derivative thereof, operably linked to a promoter and a polyadenylation recognition site. The recombinant viral vector may also include other regulatory sequences to establish expression of the Factor I, or fragment or derivative, such as a ribosome binding element, terminator, enhancer, selection marker, intron, polyA signal, and/or origin of replication, or a Woodchuck Hepatitis Virus (WHP) Post-transcriptional Regulatory Element (WPRE) sequence, or a mutated WPRE sequence.

In some embodiments, the regulatory sequences impart tissue-specific gene expression capabilities. In some cases, the tissue-specific regulatory sequences bind tissue specific transcription factors that induce transcription in a tissue specific manner. Such tissue-specific regulatory sequences (e.g., promoters, enhancers, etc.) are well known in the art.

In other embodiments, a promoter sequence that specifically expresses in a subset of one or more cells of the target tissue (for example, hepatocytes), may be used.

In some embodiments, the Factor I, or fragment or derivative thereof, is exclusively expressed in particular cells of the target tissue (for example, hepatocytes), and not in other cells of that tissue.

For example, the promoter may be a hAAT promoter.

In one embodiment, the promoter is a liver-specific promoter selected from HLP, LP1, HLP2, hAAT, HCR-hAAT, ApoE-hAAT, and LSP.

In one embodiment, the promoter comprises, or consists of, a nucleotide sequence that is at least 60%, 70%, 80%, 90%, 95%, 96%, 97%, 98%, or 99% identical over its entire length to the nucleotide sequence of SEQ ID NO: 8. In one embodiment, the promoter comprises, or consists of, the nucleotide sequence of SEQ ID NO: 8.

In one embodiment, the promoter comprises, or consists of, a nucleotide sequence that is at least 60%, 70%, 80%, 90%, 95%, 96%, 97%, 98%, or 99% identical over its entire length to the nucleotide sequence of SEQ ID NO: 9. In one embodiment, the promoter comprises, or consists of, the nucleotide sequence of SEQ ID NO: 9.

In one embodiment, the promoter comprises, or consists of, a nucleotide sequence that is at least 60%, 70%, 80%, 90%, 95%, 96%, 97%, 98%, or 99% identical over its entire length to the nucleotide sequence of SEQ ID NO: 10. In one embodiment, the promoter comprises, or consists of, the nucleotide sequence of SEQ ID NO: 10.

In some embodiments, the recombinant viral vector, or the expression cassette, comprises a liver-specific enhancer, such as liver-specific apolipoprotein E (ApoE) enhancer. In particular embodiments, the recombinant viral vector, or the expression cassette, comprises a liver-specific promoter, such as a hAAT promoter, and a liver-specific enhancer, such as liver-specific apolipoprotein E (ApoE) enhancer.

In some embodiments the expression cassette may be flanked by viral cis-acting regulatory sequences, such as inverted terminal repeats (ITRs).

In particular embodiments, the recombinant virus particle infects the liver (for example, hepatocytes) of the subject following administration, resulting in expression of the Factor I, or the fragment or derivative thereof, from the liver (for example, hepatocytes), and secretion of the Factor I, or the fragment or derivative thereof, into the subject's bloodstream. In such embodiments, the encoded Factor I, or the fragment or derivative thereof, may be expressed from a human alpha-1-anti-trypsin (hAAT) promoter.

AAV is a small, non-enveloped virus that consists of a linear single-stranded DNA genome with a packaging capacity of approximately 4.7kb. Its replicative cycle is dependent on co-infection of a helper virus that is able to complement missing genes for AAV replication (Buller, et al., J. Virol., 40(1):241-247, October 1981), for example, adenovirus (believed to be the natural helper virus) (Urabe, et al., J. Virol., 80(4):1874-1885, February 2006), herpesvirus (Afione, et al., J. Virol., 70(5):3235-3241, May 1996) or baculovirus (Samulski, et al., EMBO J., 10(12):3941-3950, December 1991). The fact that AAV is a "naturally defective" virus adds a safety barrier to prevent inappropriate spread of viral vector in clinical applications (Nakai, et al., J. Virol., 75(15):6969-6976, August 2001). Recombinant AAV (rAAV) vectors may be generated by insertion of a transgene (i.e. nucleic acid encoding Factor I, or the fragment or derivative thereof) between the two inverted terminal repeats (ITR). Recombinant AAV particles may be produced, for example, by co-transfection of such vectors with one or more plasmids encoding for all other essential genes. They can infect both dividing and quiescent cells, and persist mainly in an episomal form, as opposed to the wild-type AAV that preferably integrates at a specific site in human chromosome 19. If present in a dividing cell, the episomal AAV genome gets rapidly diluted out during cell division.

A "recombinant AAV vector (rAAV vector)" refers to a polynucleotide vector comprising nucleic acid encoding Factor I, or the fragment or derivative thereof that is flanked by at least one AAV inverted terminal repeat sequence (ITR). In particular embodiments, the nucleic acid encoding Factor I, or the fragment or derivative thereof, is flanked by two AAV ITRs. Such rAAV vectors can be replicated and packaged into infectious viral particles when present in a host cell that has been infected, for example, with a suitable helper virus (or that is expressing suitable helper functions) and another plasmid that is expressing AAV rep and cap gene products (i.e. AAV Rep and Cap proteins), as explained in more detail below.

In some embodiments, the rAAV vector comprises an expression cassette flanked by at least one AAV ITR sequence, wherein the expression cassette comprises the nucleic acid encoding Factor I, or the fragment or derivative thereof, operably linked to a promoter and a polyadenylation recognition site, and optionally a WPRE sequence or a mutant WPRE sequence. In particular embodiments, the expression cassette is flanked by two AAV ITR
sequences. In some embodiments, the expression cassette may further comprise control sequences including transcription initiation and termination sequences.

A "helper virus" for AAV refers to a virus that allows AAV (which is a defective parvovirus) to be replicated and packaged by a host cell. A number of such helper viruses have been identified, including adenoviruses, herpesviruses and poxviruses such as vaccinia. The adenoviruses encompass a number of different subgroups, although Adenovirus type 5 of subgroup C (Ad5) is most commonly used. Numerous adenoviruses of human, non-human mammalian and avian origin are known and are available from depositories such as the ATCC. Viruses of the herpes family, which are also available from depositories such as ATCC, include, for example, herpes simplex viruses (HSV), Epstein-Barr viruses (EBV), cytomegaloviruses (CMV) and pseudorabies viruses (PRV).

An "inverted terminal repeat" or "ITR" sequence is a term well understood in the art and refers to relatively short sequences found at the termini of viral genomes which are in opposite orientation. An "AAV inverted terminal repeat (ITR)" sequence, also a term well-understood in the art, is a sequence of approximately 145 nucleotides that is present at both termini of the native single-stranded AAV genome. The outermost 125 nucleotides of the ITR can be present in either of two alternative orientations, leading to heterogeneity between different AAV genomes and between the two ends of a single AAV genome. The outermost 125 nucleotides also contain several shorter regions of self-complementarity (designated A, A', B, B', C, C' and D regions), allowing intrastrand base-pairing to occur within this portion of the ITR.

The AAV ITR sequence(s) should function as intended for the rescue, replication and packaging of the AAV virion (see Davidson et al., PNAS, 2000, 97(7)3428-32; Passini et al., J. Virol., 2003, 77(12):7034-40; and Pechan et al., Gene Ther., 2009, 16:10-16). AAV ITRs for use in vectors of the invention need not have a wild-type nucleotide sequence (for example, as described in Kotin, Hum. Gene Ther., 1994, 5:793-801), and may be altered by the insertion; deletion or substitution of nucleotides, or the AAV ITRs may be derived from any of several AAV serotypes (see Gao et al., PNAS, 2002, 99(18): 11854-6; Gao et al., PNAS, 2003, 100(10):6081-6; and Bossis et al., J. Virol., 2003, 77(12):6799-810.

ITRs for use in vectors of the invention may comprise or consist of nucleotide sequence of a naturally occurring ITR, or may comprise or consist of nucleotide sequence that is at least 60%, 70%, 80%, 90%, 95%, 96%, 97%, 98%, or 99% identical over its entire length with the nucleotide sequence of a naturally occurring ITR. In particular, AAV ITRs for use in vectors of the invention may comprise or consist of nucleotide sequence of a naturally occurring AAV ITR, or may comprise or consist of nucleotide sequence that is at least 60%, 70%, 80%, 90%, 95%, 96%, 97%, 98%, or 99% identical over its entire length with the nucleotide sequence of a naturally occurring AAV ITR, such as an AAV ITR of any of the following AAV serotypes: AAV1, AAV2, AAV3, AAV4, AAV5, AAV6, AAV7, AAV8, AAV9, AAV10, AAV11, AAV12.

AAV vectors can integrate into the human genome, although the *in vivo* integration rate is low. The primary source of rAAV-mediated gene expression is extrachromosomal, not integrated genomes. In a model of murine hepatectomy, less than 10% of persistent vector genomes are integrated in the liver (Miller, et al., Nat. Genet., 30(2):147-148, February 2002).

AAV has an impressive safety record and has not been associated with any known human or animal diseases, although most humans (> 70%) are seropositive for one or more serotypes. AAV vectors have not been associated with toxicity or an inflammatory response, although the generation of neutralizing antibodies that may affect re-administration. Such immune responses may be reduced, however, by manipulating the capsid sequence (Mingozzi and High, Blood, 122(1):23-36, July 2013).

An "rAAV particle" refers to a virus particle composed of at least one AAV capsid protein and an encapsidated recombinant viral vector genome, particularly an encapsidated rAAV vector genome.

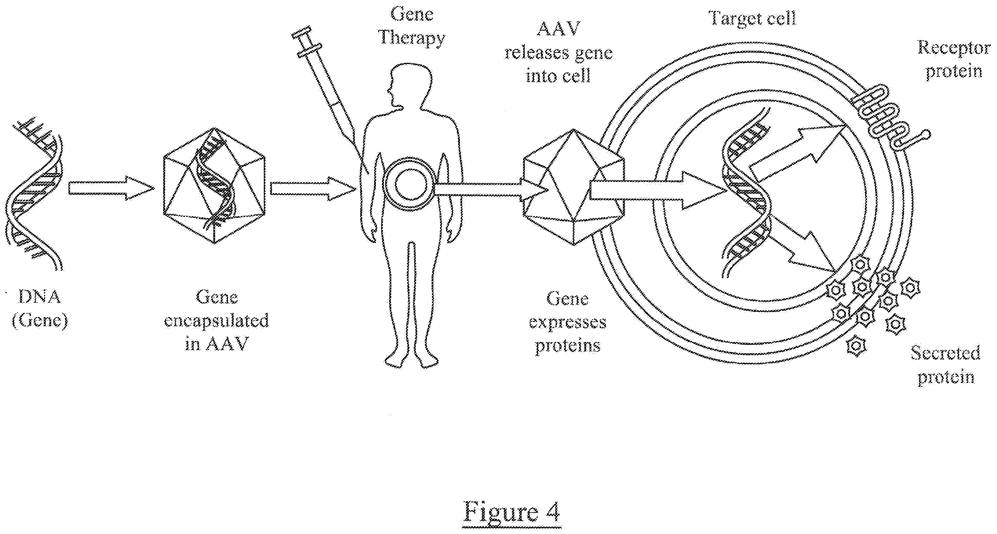

The basic steps of an AAV gene therapy method in accordance with the invention are outlined in Figure 4. A recombinant AAV vector encoding Factor I, or the fragment or derivative thereof, is used to produce rAAV particles encapsidating the vector. Following administration of the rAAV particles to a subject, for example by injection, the rAAV particles infect target cells, and the Factor I, or fragment or derivative thereof encoded by the vector is expressed by the target cell, leading to secretion of the Factor I or fragment or derivative into the subject's bloodstream. Such methods are explained in more detail below.

Heparan sulfate proteoglycan (HSPG) is known to act as the cellular receptor for AAV serotype 2 (AAV2) particles (Summerford, C. and Samulski, R.J. (1998) J. Virol. 72(2): 1438-45). Binding between an AAV2 particle and HSPG at the cell membrane serves to attach the particle to the cell. Other cell surface proteins such as fibroblast growth factor receptor and αvβ5 integrin may also facilitate cellular infection. After binding, an AAV2 particle may enter the cell through mechanisms including receptor mediated endocytosis via clathrin-coated pits. An AAV2 particle may be released from an endocytic vesicle upon endosomal acidification. This allows the AAV2 particle to travel to the perinuclear region and then the cell nucleus.

The capsid of AAV is known to include three capsid proteins: VP1, VP2, and VP3. These proteins contain significant amounts of overlapping amino acid sequence and unique N-terminal sequences. An AAV2 capsid includes 60 subunits arranged by icosahedral symmetry (Xie, Q., et al. (2002) Proc. Natl. Acad. Sci. 99(16): 10405-10). VP1, VP2, and VP3 have been found to be present in a 1:1:10 ratio. The binding between AAV2 capsid proteins and HSPG occurs via electrostatic interactions between basic AAV2 capsid protein residues and negatively charged glycosaminoglycan residues (Opie, SR et al., (2003) J. Virol. 77:6995-7006; Kern, A et al., (2003) J. Virol. 11:11072-11081). Specific capsid residues implicated in these interactions include R484, R487, K532, R585, and R588. Viruses infect their natural host cell most efficiently. Pseudotyping involves exchanging the surface proteins that mediate cell entry with the ones from another virus in order to change the viral tropism. Examples of pseudotyping include lentivirus pseudotyped with protein G of vesicular stomatitus virus (VSV G-pseudotyped lentivirus) that can transfect almost every cell (Akkina, et al., J. Virol., 70(4):2581-2585, April 1996; Willett and Bennett, Front Immunol, 4:261, 2013; Sharland, et al., Discov Med, 9(49):519-527, June 2010). In other cases, the tropism of a virus is limited to only the target cell. This allows reduction of the vector dose administrated and prevents transgene expression outside the respective cell type, for example expression only in the eye (Grimm, et al., Blood, 102(7):2412-2419, October 2003) or the liver (Thomas, et al., J. Virol., 78(6):3110-3122, March 2004; Lisowski, et al., Nature, 506(7488):382-386, February 2014; Niidome and Huang. Gene Ther., 9(24):1647-1652, December 2002). Pseudotypes can also be artificially generated, for example by preparing libraries composed of DNA-shuffled AAV capsids with improved tropism for human hepatocytes (Li and Huang. Gene Ther., 13(18):1313-1319, September 2006). This can help to overcome low vector uptake and transgene expression.

According to the invention the recombinant virus particle may be pseudotyped to confer tropism for the cell type(s) to be infected. For example, in embodiments of the invention in which the recombinant virus particle infects liver cells (for example hepatocytes), the recombinant virus particle is pseudotyped to confer liver tropism, particularly hepatocyte tropism.

The recombinant virus particle may be an rAAV particle pseudotyped to confer hepatocyte tropism.

Over one hundred different AAV serotypes have been identified (Atchison, et al., Science, 149(3685):754-756, August 1965; Warrington, et al., J. Virol., 78(12):6595-6609, June 2004). Pseudotyping can be easily achieved for rAAV virus particles for example, by packaging the capsid sequence of another serotype into a helper plasmid. Several approaches to improve tropism and transgene expression may be taken: i) construction of capsid libraries that consist of randomized capsid sequences (Li and Huang, Gene Ther., 13(18):1313-1319, September 2006); ii) insertion of amino acid sequences into the capsid of AAV2 (up to 30 kDa, possibility to insert a ScFv sequence to target AAV to a specific cell type) (Calcedo, et al., J. Infect. Dis., 199(3):381-390, February 2009); and iii) a rational design approach by combining knowledge of delivery mechanisms with AAV structural analyses (Calcedo, et al., Clin. Vaccine Immunol., 18(9):1586-1588, September 2011).

In some embodiments, an rAA V vector is a vector derived from an AAV serotype, including AAV1, AAV2, AAV3, AAV4, AAV5, AAV6, AAV7, AAV8, AAV9, AAV10, AAV11, or AAV12 ITRs. An rAAV particle may comprise capsid protein derived from any AAV serotype, including AAV1, AAV2, AAV3, AAV4, AAV5, AAV6, AAV7, AAV8, AAV9, AAV10, AAVrh10, AAV11, or AAV12 capsid. Different AA V serotypes are used to optimize transduction of particular target cells or to target specific cell types within a particular target tissue (e.g., a liver tissue). An rAAV particle can comprise viral proteins and viral nucleic acids of the same serotype or a mixed serotype.

A capsid protein of a recombinant virus particle of the invention may comprise or consist of amino acid sequence of a naturally occurring capsid protein (for example, a naturally occurring AAV capsid protein, such as a capsid protein of AAV serotype AAV1, AAV2, AAV3, AAV4, AAV5, AAV6, AAV7, AAV8, AAV9, AAV10, AAVrh10, AAV11, or AAV12), or may be a derivative of a naturally occurring capsid protein that includes one or more amino acid substitutions, deletions, or additions, compared with the amino acid sequence of the naturally occurring capsid protein, for example to confer enhanced tropism for a desired tissue type or cell type (such as hepatocyte tropism), or to reduce the immunogenicity of the recombinant virus particle.

In some embodiments a capsid protein of a recombinant virus particle of the invention has an amino acid sequence that has at least 60%, 70%, 80%, 90%, 95%, 96%, 97%, 98%, or 99% amino acid identity along its entire length to the amino acid sequence of a naturally occurring capsid protein. In some embodiments an AAV capsid protein of a rAAV particle of the invention has an amino acid sequence that has at least 60%, 70%, 80%, 90%, 95%, 96%, 97%, 98%, or 99% amino acid identity along its entire length to the amino acid sequence of a naturally occurring AAV capsid protein, for example a naturally occurring AAV capsid protein of serotype AAV1, AAV2, AAV3, AAV4, AAV5, AAV6, AAV7, AAV8, AAV9, AAV10, AAVrh10, AAV11, or AAV12.

In some embodiments a capsid protein of a recombinant virus particle of the invention is a non-naturally occurring capsid protein, for example a non-naturally occurring AAV capsid protein, such as an engineered or chimeric capsid protein.

Chimeric capsid proteins include those generated by recombination between two or more capsid coding sequences of naturally occurring AAV serotypes. This may be performed for example by a marker rescue approach in which non-infectious capsid sequences of one serotype are co-transfected with capsid sequences of a different serotype, and directed selection is used to select for capsid sequences having desired properties. The capsid sequences of the different serotypes can be altered by homologous recombination within the cell to produce novel chimeric capsid proteins.

Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence.

Examples of non-naturally occurring capsid proteins suitable for use in the present invention are described in WO 2016/181123 and WO 2013/029030.

In one embodiment, a capsid protein of a recombinant virus particle of the invention is a MutC capsid protein (for example, a Mut C capsid protein as described in WO 2016/181123). In one embodiment, a capsid protein of a recombinant virus particle of the invention has an amino acid sequence that has at least 60%, 70%, 80%, 90%, 95%, 96%, 97%, 98%, or 99% amino acid identity along its entire length to the amino acid sequence of SEQ ID NO: 11. In one embodiment, a capsid protein of a recombinant virus particle of the invention has an amino acid sequence that comprises, or consists of, the amino acid sequence of SEQ ID NO: 11.

In one embodiment, a capsid protein of a recombinant virus particle of the invention is an LK03 capsid protein (for example, an LK03 capsid protein as described in WO 2013/029030). In one embodiment, a capsid protein of a recombinant virus particle of the invention has an amino acid sequence that has at least 60%, 70%, 80%, 90%, 95%, 96%, 97%, 98%, or 99% amino acid identity along its entire length to the amino acid sequence of SEQ ID NO: 12. In one embodiment, a capsid protein of a recombinant virus particle of the invention has an amino acid sequence that comprises, or consists of, the amino acid sequence of SEQ ID NO: 12.

In one embodiment, a capsid protein of a recombinant virus particle of the invention is an AAVrh10 protein as described in Wang et al. Mol Ther. 2015 Dec; 23(12): 1877-1887 or a derivative thereof that has at least 60%, 70%, 80%, 90%, 95%, 96%, 97%, 98%, or 99% amino acid identity along its entire length to the amino acid sequence of the disclosed AAVrh10 protein.

In particular embodiments of the invention, the recombinant viral vector is an AAV2-derived viral vector, i.e. a recombinant viral vector in which the nucleic acid encoding Factor I, or the fragment or derivative thereof, is flanked by at least one AAV2 ITR sequence (Kotterman and Schaffer, Nat. Rev. Genet., 15(7):445-451, July 2014), or a derivative thereof with a nucleotide sequence that has at least 60%, 70%, 80%, 90%, 95%, 96%, 97%, 98%, or 99% identity over its entire length with the nucleotide sequence of a naturally occurring AAV2 ITR. In some embodiments, the nucleic acid encoding Factor I, or the fragment or derivative thereof, is flanked by two AAV2 ITRs, or AAV2 ITR derivatives (wherein each AAV2 ITR derivative comprises nucleotide sequence that has at least 60%, 70%, 80%, 90%, 95%, 96%, 97%, 98%, or 99% identity over its entire length with the nucleotide sequence of a naturally occurring AAV2 ITR).

AAV2 has a broad tropism and transduces many cell types, including hepatocytes, relatively efficiently *in vivo.* Pseudotyping a recombinant AAV vector with different capsid proteins can dramatically alter the tropism. Both AAV8 and AAV9 have higher affinities for hepatocytes when compared to AAV2. In particular, AAV8 can transduce three- to fourfold more hepatocytes and deliver three- to fourfold more genomes per transduced cell when compared to AAV2.

In one embodiment, the recombinant viral vector is not an AAV2-derived viral vector.

In some embodiments, an rAAV particle of the invention comprises AAV8 or AAV9 capsid protein (or a derivative thereof that has at least 60%, 70%, 80%, 90%, 95%, 96%, 97%, 98%, or 99% amino acid identity along its entire length to the amino acid sequence of a naturally occurring AAV8 or AAV9 capsid protein). In particular embodiments, the rAAV particle encapsidates an AAV2-derived viral vector, and is pseudotyped with AAV8 capsid (rAAV2/8) or AAV9 capsid (rAAV2/9) (or a derivative thereof that has at least 60%, 70%, 80%, 90%, 95%, 96%, 97%, 98%, or 99% amino acid identity along its entire length to the amino acid sequence of a naturally occurring AAV8 or AAV9 capsid protein). In other embodiments, the rAAV particle encapsidates an AAV2-derived viral vector, and is pseudotyped with Mut C capsid (rAAV2/Mut C) or LK03 capsid (rAAV2/LK03) or AAVrh10 capsid (rAAV2/rh10).

Numerous methods are known in the art for production of rAAV particles, including transfection, stable cell line production, and infectious hybrid virus production systems which include adenovirus-AAV hybrids, herpesvirus-AAV hybrids (Conway, et al., (1997) J. Virology 71(11):8780-8789) and baculovirus-AAV hybrids.

rAAV production cultures for the production of rAAV virus particles all require: i) suitable host cells, including, for example, human-derived cell lines such as HeLa, A549, 293, or 293T cells, or insect-derived cell lines such as SF-9, in the case of baculovirus production systems; ii) suitable helper function, provided by wild-type or mutant adenovirus (such as temperature sensitive adenovirus), herpes virus, baculovirus, or a plasmid construct providing helper functions; iii) AAV rep and cap genes; iv) nucleic acid encoding Factor I, or a fragment or derivative thereof, flanked by at least one AAV ITR sequence; and v) suitable media and media components to support rAAV production.

Suitable media known in the art may be used for the production of rAAV particles. These media include, without limitation, media produced by Hyclone Laboratories and JRH including Modified Eagle Medium (MEM), Dulbecco's Modified Eagle Medium (DMEM) high glucose, custom formulations such as those described in US 6,566,118, and Sf-900 II SFM media as described in US 6,723,551.

The rAAV particles can be produced using methods known in the art. See, for example, US 6,566,118, US 6,989,264, and US 6,995,006. In practicing the invention, host cells for producing rAAV particles include mammalian cells, insect cells, plant cells, microorganisms and yeast. Host cells can also be packaging cells in which the AAV rep and cap genes are stably maintained in the host cell, or producer cells in which the AAV vector genome is stably maintained. Exemplary packaging and producer cells are derived from 293, 293T, A549 or HeLa cells. AAV vectors are purified and formulated using standard techniques known in the art.

In some embodiments, rAAV particles may be produced by a triple transfection method, such as the exemplary triple transfection method provided *infra.* Briefly, a plasmid containing a rep gene and a capsid gene, a helper adenoviral plasmid, and a plasmid comprising the transgene (i.e. the gene encoding Factor I, or the fragment or derivative thereof), may be transfected (e.g., using the calcium phosphate method, or Polyethylenimine) into a cell line (e.g., HEK-293 cells), and virus may be collected and optionally purified.

In some embodiments, rAAV particles may be produced by a producer cell line method, such as the exemplary producer cell line method provided *infra* (see also referenced in Martin et al., (2013) Human Gene Therapy Methods 24:253-269). Briefly, a cell line (e.g., a HeLa cell line) may be stably transfected with a plasmid containing a rep gene, a capsid gene, and a promoter-transgene sequence. Cell lines may be screened to select a lead clone for rAAV production, which may then be expanded to a production bioreactor and infected with an adenovirus (e.g., a wild-type adenovirus) as helper to initiate rAAV production. Virus may subsequently be harvested, adenovirus may be inactivated (e.g., by heat) and/or removed, and the rAAV particles may be purified.

Described herein is a method is provided for producing an rAAV particle of the invention comprising: (a) culturing a host cell under conditions for production of rAAV particles, wherein the host cell comprises (i) one or more AAV packaging genes, wherein each AAV packaging gene encodes an AAV replication and/or encapsidation protein; (ii) an rAAV vector comprising nucleic acid encoding Factor I, or a fragment or derivative thereof that retains C3b-inactivating and iC3b-degradation activity, flanked by at least one AAV ITR (preferably two AAV ITRs), and (iii) an AAV helper function (i.e. genes required for a productive AAV life cycle, for example from adenovirus, herpesvirus, or baculovirus); and (b)recovering the rAAV particles produced by the host cell. In some embodiments, (i), (ii), and (iii) may be provided on three separate plasmids, for example as described in Example 1 below.

In some embodiments, the, or each AAV ITR comprises or consists of nucleotide sequence of a naturally occurring AAV ITR, for example any of the following serotype AAV ITRs: AAV1, AAV2, AAV3, AAV4, AAV5, AAV6, AAV7, AAV8, AAV9, AAV 10, AAV11, AAV12, for example, AAV2 ITR, or comprises or consists of a nucleotide sequence that is at least 60%, 70%, 80%, 90%, 95%, 96%, 97%, 98%, or 99% identical over its entire length with the nucleotide sequence of a naturally occurring AAV ITR, such as an AAV ITR of any of the following AAV serotypes: AAV1, AAV2, AAV3, AAV4, AAV5, AAV6, AAV7, AAV8, AAV9, AAV 10, AAV11, AAV12.

In some embodiments, the encapsidation protein is an AAV8 or AAV9 or Mut C or LK03 or AAVrh10 encapsidation protein.

Suitable rAAV production culture media may be supplemented with serum or serum-derived recombinant proteins at a level of 0.5%-20% (v/v or w/v). Alternatively, as is known in the art, rAAV particles may be produced in serum-free conditions which may also be referred to as media with no animal-derived products. One of ordinary skill in the art may appreciate that commercial or custom media designed to support production of rAAV particles may also be supplemented with one or more cell culture components know in the art, including without limitation glucose, vitamins, amino acids, and/or growth factors, in order to increase the titre of rAAV in production cultures.

rAAV production cultures can be grown under a variety of conditions (over a wide temperature range, for varying lengths of time, and the like) suitable to the particular host cell being utilized. As is known in the art, rAAV production cultures include attachment-dependent cultures which can be cultured in suitable attachment-dependent vessels such as, for example, hyperflasks, roller bottles, hollow fiber filters, microcarriers, and packed-bed or fluidized-bed bioreactors. rAAV vector production cultures may also include suspension-adapted host cells such as HeLa, 293, 293T, and SF-9 cells which can be cultured in a variety of ways including, for example, spinner flasks, stirred tank bioreactors, and disposable systems such as the Wave bag system.

rAAV particles of the invention may be harvested from rAAV production cultures by lysis of the host cells of the production culture or by harvest of the spent media from the production culture, provided the cells are cultured under conditions known in the art to cause release of rAAV particles into the media from intact cells, as described more fully in US 6,566,118. Suitable methods of lysing cells are also known in the art and include for example multiple freeze/thaw cycles, sonication, microfluidization, and treatment with chemicals, such as detergents and/or proteases.

In a further embodiment, the rAAV particles are purified. The term "purified" as used herein includes a preparation of rAAV particles devoid of at least some of the other components that may also be present where the rAAV particles naturally occur or are initially prepared from. Thus, for example, isolated rAAV particles may be prepared using a purification technique to enrich them from a source mixture, such as a culture lysate or production culture supernatant. Enrichment can be measured in a variety of ways, such as, for example, by the proportion of DNase-resistant particles (DRPs) or genome copies (gc) present in a solution, or by infectivity, or it can be measured in relation to a second, potentially interfering substance present in the source mixture, such as contaminants, including production culture contaminants or in-process contaminants, including helper virus, or media components.

In some embodiments, the rAAV production culture harvest is clarified to remove host cell debris. In some embodiments, the production culture harvest is clarified by filtration through a series of depth filters including, for example, a grade DOHC Millipore Millistak+HC Pod Filter, a grade A1HC Millipore Millistak+ HC Pod Filter, and a 0.2µm Filter Opticap XL1O Millipore Express SHC Hydrophilic Membrane filter. Clarification can also be achieved by a variety of other standard techniques known in the art, such as, centrifugation or filtration through any cellulose acetate filter of 0.2µm or greater pore size known in the art.

In some embodiments, the rAAV production culture harvest is further treated with Benzonase^{®} to digest any high molecular weight DNA present in the production culture. In some embodiments, the Benzonase^{®} digestion is performed under standard conditions known in the art including, for example, a final concentration of 1-2.5 units/ml of Benzonase^{®} at a temperature ranging from ambient to 37°C for a period of 30 minutes to several hours.

rAAV particles may be isolated or purified using one or more of the following purification steps: density gradient ultracentrifugation using caesium chloride (e.g. as described in Cooper et al., Molecular Therapy (2005) 11, Supplement 1, S53-S54), or iodixanol; equilibrium centrifugation; flow-through anionic exchange filtration; tangential flow filtration (TFF) for concentrating the rAAV particles; rAAV capture by apatite chromatography; heat inactivation of helper virus; rAAV capture by hydrophobic interaction chromatography; buffer exchange by size exclusion chromatography (SEC); nanofiltration; and rAAV capture by anionic exchange chromatography, cationic exchange chromatography, or affinity chromatography. These steps may be used alone, in various combinations, or in different orders. Methods to purify rAAV particles are described, for example, in Xiao et al., (1998) Journal of Virology 72:2224-2232; US 6,989,264; US 8,137,948; and WO 2010/148143.

The recombinant virus particle may be administered to the subject by any suitable route. In particular embodiments, the recombinant virus particle is administered intravenously to the subject.

In particular embodiments of the invention, the recombinant virus particle is not administered to the subject via local (or topical) administration.

In particular embodiments of the invention, the recombinant virus particle is not administered to the eye. Thus, in particular embodiments, administration of the recombinant virus particle does not comprise intraocular administration, such as intraocular, for example sub-retinal, injection.

A recombinant virus particle of the invention may be administered to the subject via systemic administration.

A skilled person will be familiar with multiple routes by which systemic administration may be achieved, and associated techniques.

By way of example, systemic administration may achieved via parenteral administration. Examples of parenteral administration include: intravenous administration, intra-arterial administration, intramuscular administration, intrathecal administration, and subcutaneous administration.

Thus, in particular embodiments, a recombinant virus particle of the invention is administered to the subject systemically.

A preferred route of administration of a recombinant virus particle of the invention is intravenous administration.

In one embodiment, a recombinant virus particle of the invention is administered into the hepatic portal vein of the subject. Advantageously, administration of the recombinant virus particle into the hepatic portal vein directs the recombinant virus particle to the liver.

As described above, in particular embodiments, the recombinant virus particle infects the liver (for example, hepatocytes) of the subject following administration, resulting in expression of the Factor I, or the fragment or derivative thereof, from the liver (for example, hepatocytes), and secretion of the Factor I, or the fragment or derivative thereof, into the subject's bloodstream.

The Factor I, or a fragment or derivative thereof, secreted into the subject's bloodstream from the liver may provide a therapeutic effect in a target tissue or cell located elsewhere in the body. The Factor I, or a fragment or derivative thereof, secreted into the subject's bloodstream from the liver may provide a systemic increase in the level of C3b-inactivating and iC3b-degradation activity in the subject.

AAV-mediated liver-directed gene therapy is discussed in Sands Methods Mol Biol. 2011; 807: 141-157, and Sharland et al., Discovery Medicine, 9(49):519-527, June 2010.For example, depending on the dose, AAV8 can transduce up to 90-95% of hepatocytes in the mouse liver following intraportal vein injection. Interestingly, comparable levels of transduction can be achieved following intravenous injection. Direct intraparenchymal injection of an AAV vector also mediates relatively high-level long-term expression. Additional specificity can be conferred by using liver-specific promoters in conjunction with AAV8 capsid proteins.

An effective amount of recombinant viral vector (in some embodiments encapsidated by recombinant virus particles) is administered, depending on the objectives of treatment. For example, where a low percentage of transduction can achieve the desired therapeutic effect, then the objective of treatment is generally to meet or exceed this level of transduction. In some instances, this level of transduction can be achieved by transduction of only about 1 to 5% of the target cells, in some embodiments at least about 20% of the cells of the desired
tissue type, in some embodiments at least about 50%, in some embodiments at least about 80%, in some embodiments at least about 95%, in some embodiments at least about 99% of the cells of the desired tissue type.

In some embodiments of the invention, the dose of viral particles administered to the subject is 1 x 10⁸ to 1 x 10¹³ genome copies/kg of body weight.

In some embodiments of the invention, the total amount of viral particles administered to the subject is 1 x 10⁹ to 1 x 10¹⁵ genome copies.

For embodiments in which the virus particles are administered to the subject, as a guide, the number of virus particles administered per injection is generally 1 x 10⁶ to 1 x 10¹⁴ particles, 1 x 10⁷ to 1 x 10¹³ particles, 1 x 10⁹ to 1 x 10¹² particles, or 1 x 10¹¹ particles.

The recombinant virus particles may be administered by one or more injections, either during the same procedure or spaced apart by days, weeks, months, or years. In some embodiments, multiple vectors may be used to treat the subject.

In some embodiments, at least 5%, 10%, 15%, 20%, 25%, 30%, 35%, 40%, 45%, 50%, 55%, 60%, 65%, 70%, or 75%, up to 100% of cells of the target tissue (for example, hepatic cells of the liver) are transduced. In some embodiments, 5% to 100%, 10% to about 50%, 10% to 30%, 25% to 75%, 25% to 50%, or 30% to 50% of cells of the target tissue (for example, hepatic cells of the liver) are transduced.

Methods to identify cells transduced by recombinant viral particles comprising a recombinant virus particle capsid are known in the art. For example, immunohistochemistry or the use of a marker such as enhanced green fluorescent protein can be used to detect transduction of recombinant virus particles.

In some embodiments, the recombinant virus particles are administered (for example by injection) to one or more locations in the desired tissue (for example, the liver). In some embodiments, the recombinant virus particles are administered (for example by injection) to any one of one, two, three, four, five, six, seven, eight, nine, ten or more than ten locations in the tissue.

In some embodiments, the recombinant virus particles are administered to more than one location simultaneously or sequentially. In some embodiments, multiple injections of recombinant virus particles are no more than one hour, two hours, three hours, four hours, five hours, six hours, nine hours, twelve hours or 24 hours apart.

The subject may be administered with one or more additional therapeutic agents for treating complement-mediated disorders. Such agent(s) may be co-administered with the virus particles, or administered sequentially. The interval between sequential administration can be in terms of at least (or, alternatively, less than) minutes, hours, or days.

It will be appreciated that the C3b-inactivating and iC3b-degradation activity in the subject following expression of the Factor I, or fragment or derivative thereof, from the nucleic acid of the recombinant viral vector may comprise C3b-inactivating and iC3b-degradation activity from the subject's endogenous Factor I (i.e. the subject's Factor I not produced by expression from the recombinant viral vector) and C3b-inactivating and iC3b-degradation activity produced by expression from the recombinant viral vector, such that the total level of C3b-inactivating and iC3b-degradation activity in the subject is increased (relative to the level prior to administration of the vector) or exceeds a normal level.

The level of C3b-inactivating and iC3b-degradation activity in the subject may be the level in serum of the subject. For a human subject, the normal level of C3b-inactivating and iC3b-degradation activity is equivalent to that provided by 30-40µg/ml Factor I in serum of the subject.

Prior to administration of a recombinant virus particle according to the invention, the subject may have a normal level of endogenous Factor I. In other embodiments, the subject may have lower than normal levels of Factor I, for example the Factor I in serum of the subject may be less than 30µg/ml and greater than 0µg/ml.

By way of further example, the level of Factor I in serum of the subject may be less than 25µg/ml and greater than 0µg/ml, or less than 20µg/ml and greater than 0µg/ml, or less than 15µg/ml and greater than 0µg/ml, or less than 10µg/ml and greater than 0µg/ml, or less than 5µg/ml and greater than 0µg/ml.

In particular embodiments of the invention, the level of C3b-inactivating and iC3b-degradation activity in the subject is increased to a level that exceeds a normal level.

In particular embodiments of the invention, the level of C3b-inactivating and iC3b-degradation activity in the subject is increased to a level that is at least 5%, 10%, 15%, 20%, or 25% above the normal level.

In particular embodiments of the invention, the level of C3b-inactivating and iC3b-degradation activity in the subject is increased to a level that is up to twice the normal level, or up to 80%, 60%, 40%, or 20% above the normal level.

For example, the level of C3b-inactivating and iC3b-degradation activity in the subject may be increased to a level that is 5-100%, 5-80%, 5-60%, 5-40%, 5-20%, 10-100%, 10-80%, 10-60%, 10-40%, 10-20%, 15-100%, 15-80%, 15-60%, 15-40%, 15-20%, 20-100%, 20-80%, 20-60%, 20-40%, 25-100%, 25-80%, 25-60%, or 25-40% above the normal level.

As described above, the subject may have a level of endogenous Factor I in serum that is lower than a normal level. Thus, the level of C3b-inactivating and iC3b-degradation activity in such a subject prior to administration of a recombinant virus particle according to the invention may be below a normal level. Therapeutic effects may be obtained in such a subject by increasing the level of C3b-inactivating and iC3b-degradation activity to a level that is greater than the baseline level of C3b-inactivating and iC3b-degradation activity in the subject prior to administration of a recombinant virus particle according to the invention. Optionally, said increased level of C3b-inactivating and iC3b-degradation activity may remain below a normal level.

Thus, in particular embodiments of the invention, the level of C3b-inactivating and iC3b-degradation activity in the subject is increased to a level that is greater than the baseline level in the subject prior to administration of a recombinant virus particle according to the invention.

In particular embodiments of the invention, the level of C3b-inactivating and iC3b-degradation activity in the subject is increased to a level that is at least 5%, 10%, 15%, 20%, or 25% above the baseline level in the subject prior to administration of a recombinant virus particle according to the invention.

In particular embodiments of the invention, the level of C3b-inactivating and iC3b-degradation activity in the subject is increased to a level that is up to twice the baseline level in the subject prior to administration of a recombinant virus particle according to the invention; or up to 80%, 60%, 40%, or 20% above the baseline level in the subject prior to administration of a recombinant virus particle according to the invention.

For example, the level of C3b-inactivating and iC3b-degradation activity in the subject may be increased to a level that is 5-100%, 5-80%, 5-60%, 5-40%, 5-20%, 10-100%, 10-80%, 10-60%, 10-40%, 10-20%, 15-100%, 15-80%, 15-60%, 15-40%, 15-20%, 20-100%, 20-80%, 20-60%, 20-40%, 25-100%, 25-80%, 25-60%, or 25-40% above the baseline level in the subject prior to administration of a recombinant virus particle according to the invention.

The complement-mediated disorder may be a disorder associated with a defect in alternative pathway regulation, and in particular with over-activity of the complement C3b feedback cycle.

The complement-mediated disorder may be a disorder characterised by symptoms that are ameliorated by increased levels of C3b-inactivating and iC3b-degradation activity in the subject.

Examples of complement-mediated disorders that may be prevented or treated according to the invention include age-related macular degeneration (AMD) (particularly early (dry) AMD, or geographic atrophy), dense deposit disease (DDD), atypical haemolytic uraemic syndrome (aHUS), C3 glomerulopathies, membranoproliferative glomerulonephritis Type 2 (MPGN2 or MPGN type II), atherosclerosis, chronic cardiovascular disease, Alzheimer's disease, systemic vasculitis, paroxysmal nocturnal haemoglobinuria (PNH), and inflammatory or autoinflammatory diseases of old age.

In one embodiment, the formation of geographic atrophy is prevented or reduced. In another embodiment, the amount of geographic atrophy is reduced.

C3 glomerulopathies that may be prevented or treated according to the invention include C3 glomerulonephritis and Dense Deposit Disease (also known as membranoproliferative glomerulonephritis (MPGN) type II).

Further examples of complement-mediated disorders that may be prevented or treated according to the invention include membranoproliferative glomerulonephritis type I (MPGN type I), membranoproliferative glomerulonephritis type III (MPGN type III), Guillain-Barré syndrome, Henoch-Schonlein purpura, IgA nephropathy, and membranous glomerulonephritis. Membranoproliferative glomerulonephritis (MPGN) is also known as mesangiocapillary glomerulonephritis.

In particular embodiments of the invention, the complement-mediated disorder that is prevented, treated or ameliorated according to the invention is selected from DDD, aHUS, C3 glomerulopathies, atherosclerosis, chronic cardiovascular disease, Alzheimer's disease, systemic vasculitis, PNH, inflammatory or autoinflammatory diseases of old age, MPGN type I, MPGN type III, Guillain-Barré syndrome, Henoch-Schonlein purpura, IgA nephropathy, and membranous glomerulonephritis.

In one embodiment, said preventing, treating, or ameliorating a complement-mediated disorder in a subject in need thereof comprises reducing said subject's required dose and/or frequency of administration of a pre-existing treatment regimen, for example reducing said subject's required dose and/or frequency of administration of an inhibitor of a complement pathway component (such as an inhibitor of complement protein C5, for example an anti-C5 antibody) or of an anti-inflammatory agent (such as a steroid).

In particular embodiments of the invention, the complement-mediated disorder that is prevented or treated according to the invention is not an ocular disorder.

In particular embodiments of the invention, the complement-mediated disorder that is prevented or treated according to the invention is not a complement-mediated disorder of the eye.

In particular embodiments of the invention, the complement-mediated disorder that is prevented or treated according to the invention is not age-related macular degeneration (AMD) or geographic atrophy.

In particular embodiments of the invention, the complement-mediated disorder that is prevented or treated according to the invention is not diabetic retinopathy.

The subject may be at risk of developing a complement-mediated disorder. For example, the subject may be homozygous or heterozygous susceptible for one or more SNPs associated with the complement-mediated disorder.

In particular embodiments, the subject is at risk of developing AMD. For example, the subject may be homozygous or heterozygous susceptible for one or more SNPs associated with AMD. Examples of such SNPs include rs1061170 (encoding Y402H) of Factor H, rs800292 (encoding V62I) of Factor H, rs641153 (encoding R32Q) of Factor B, or rs2230199 (encoding R102G)of C3, and in particular rs1061170 of Factor H, rs800292 of Factor H, or rs2230199 of C3. Other examples of rare mutations in *CFI* associated with advanced AMD, which commonly result in reduced serum Factor I levels, are described by Kavanagh et al., Hum Mol Genet. 2015 Jul 1;24(13):3861-70. They include the following SNPs: rs144082872 (encoding P50A); 4:110687847 (encoding P64L); rs141853578 (encoding G119R); 4:110685721 (encoding V152M); 4:110682846 (encoding G162D); 4:110682801 (encoding N177I); rs146444258 (encoding A240G); rs182078921 (encoding G287R); rs41278047 (encoding K441R); rs121964913 (encoding R474).

Methods of the invention may further comprise determining whether the subject is at risk of developing a complement-mediated disorder (for example AMD), for example by determining whether the subject is homozygous or heterozygous susceptible for one or more SNPs associated with the complement-mediated disorder (for example, by determining whether the subject is homozygous or heterozygous susceptible for one or more of the SNPs associated with AMD listed above).

In the study described in Lay et al. (Clin Exp Immunol 181(2):314-322 Aug 2015), sera from a large panel of normal subjects were typed for three common polymorphisms, one in C3 (R102G) and two in Factor H (V62I and Y402H) that influence predisposition to AMD and to some forms of kidney disease. Three groups of sera were tested; those that were homozygous for the three risk alleles; those that were heterozygous for all three; and those homozygous for the low risk alleles. These groups vary in their response to the addition of exogenous Factor I when the alternative complement pathway is activated by zymosan. Both the reduction in the maximum amount of iC3b formed and the rate at which the iC3b is converted to C3dg are affected. For both reactions the at-risk complotype requires higher doses of Factor I to produce similar down-regulation. Since iC3b reacting with the complement receptor CR3 is a major mechanism by which complement activation gives rise to inflammation, the breakdown of iC3b to C3dg can be seen to have major significance for reducing complement induced inflammation. These findings demonstrate for the first time that sera from subjects with different complement alleles do behave as predicted in an in-vitro assay of the down-regulation of the alternative complement pathway by increasing the concentration of Factor I. These results support the use of exogenous Factor I as a therapeutic for down-regulating hyperactivity of the C3b feedback cycle and thereby providing a treatment for AMD and some forms of kidney disease.

In the study reported in Lachmann et al. (Clinical & Experimental Immunology, Volume 183, Issue 1, pages 150-156, January 2016), the amounts of Factor I that were needed to convert the highest risk group (homozygous for three AMD risk alleles, C3 (R102G), Factor H (V621 and Y402H)) to the activity of the least risk group (homozygous for the low risk alleles) was about 22µg/mi of Factor I, the reduction of the highest risk group to the heterozygous group (heterozygous for all three risk alleles) was 12.5µg/ml, and of the heterozygous group to the least risk group was 5µg/ml.

In particular embodiments of the invention, the level of C3b-inactivating and iC3b-degradation activity in the subject is increased to a level that is at least 10% above the normal level if the subject is heterozygous susceptible for one or more SNPs associated with AMD. In particular embodiments, the SNPs associated with AMD are rs1061170 of Factor H, rs800292 of Factor H, or rs2230199 of C3.

In particular embodiments of the invention, the level of C3b-inactivating and iC3b-degradation activity in the subject is increased to a level that is at least 50% above the normal level if the subject is homozygous susceptible for one or more SNPs associated with AMD. In particular embodiments, the SNPs associated with AMD are rs1061170 of Factor H, rs800292 of Factor H, or rs2230199 of C3.

Methods of the invention may further comprise determining the level of C3b-inactivating and iC3b-degradation activity in the subject at least one, two, three, four five, or six days, at least one, two, or three weeks, or at least one, two, three, four, five, or six months, or at least a year, following the administration, and repeating the administration if the level of activity is found to be at, or below the normal level. If the level of activity is found to have remained above the normal level for one year since the first administration, subsequent checks may then be made annually to confirm that the level of activity continues to remain above normal.

*In vivo,* complement Factor I (FI) is mainly expressed in the liver by hepatocytes, although it has also been found to be expressed *in vitro* in monocytes, fibroblasts, keratinocytes, and human umbilical vein endothelial cells. FI is a heterodimer, consisting of a heavy and a light chain, which are covalently linked by a disulphide bond. FI is expressed as a single pro-peptide chain. Post-translationally, the protein undergoes several modifications, including N-glycosylation in the endoplasmic reticulum and Golgi. Each chain can be glycosylated at three asparagine residues; these heavy weight glycan sugar molecules make 20-25% of the apparent protein molecular weight. Additionally, FI is proteolytically cleaved in the trans-Golgi network by furin (or paired basic amino acid cleaving enzyme, PACE), which cuts out four positively charged amino acids, namely RRKR. These four amino acids are found at the interface between the heavy and light chain and form the linker peptide. After their removal, the pro-enzyme is processed into the enzymatically active, cleaved heterodimeric form. Although pro-FI contains 28 paired basic amino acid residues throughout its sequence, furin recognizes and cleaves only at the Arg-Arg-Lys-Arg site. FI has an 18-residue N-terminal leader sequence that is cleaved off before secretion and the mature protein is released into the blood stream (Nilsson, et al., Mol. Immunol., 44(8):1835-1844, March 2007).

FI consists of several domains (Nilsson et al., 2011, Mol. Immunol. 48(14):1611-1620). The heavy chain consists of an N-terminal FI membrane attack complex domain (FIMAC), a scavenger receptor cysteine-rich domain (SRCR, also known as a CD5 domain), a low-density lipoprotein receptor 1 and 2 domain, and a small region of unknown homology, sometimes called the D-region. The light chain of FI is the enzymatically active domain, which consists of a chymotrypsin-like serine protease (SP) domain containing the residues that form the His-Asp-Ser catalytic triad.

It has been suggested that the heavy chain of Factor I binds to the substrate and orients the SP domain of intact FI towards the two cleavage sites in C3b, which are cleaved to form iC3b. In 2011, the crystal structure of FI was published and shed more light on the arrangement of the heavy and light chain in the C3b-FH-FI complex (Tsiftsoglou and Sim, J. Immunol., 173(1):367-375, July 2004). It was suggested that the substrate (i.e. the C3b-FH complex) induces structural remodelling in the active site of Factor I. This is further supported by the finding that the protease inhibitor diisopropyl fluorophosphate is only able to react with the active site serine if FI is pre-incubated with C3b. Superimposition of FI on the crystal structure of C3b-FH(1-4) complex shows that apart from the SRCR domain, the heavy chain is closely packed against C3b and cofactor. This binding abrogates the allosteric inhibitory effects of the heavy chain and induces remodelling of the SP domain, which then becomes active and cleaves C3b. The importance of the FIMAC and FI domain accessibility in proper FI function was elegantly demonstrated in a mutagenesis series (Schlott, et al., J. Mol. Biol., 318(2):533-546, April 2002).

Factor I, or the fragment or derivative thereof, encoded by a recombinant viral vector of the invention may include the native 18 amino acid residue signal sequence to ensure correct processing and secretion of the mature Factor I. Alternatively a heterologous signal sequence (i.e. a signal sequence not encoded by the native gene) may be used, or the signal sequence may be omitted.

In particular embodiments, the Factor I is human Factor I, for example human Factor I with an amino acid sequence of SEQ ID NO: 2 (variant 2, with 18-residue N-terminal signal sequence, NCBI Reference Sequence: NM_000204.4), or SEQ ID NO: 4 (variant 2, without 18-residue N-terminal signal sequence).

In other embodiments, other variants of Factor I may be used, such as human Factor I, variant 1 (this is a longer isoform, and includes an alternate in-frame exon in the central coding region compared to variant 2). The sequence of this variant is NCBI Reference Sequence: NM_001318057.1, and may be used with or without the 18-amino acid residue N-terminal signal sequence.

The fragment or derivative of Factor I may be a polypeptide that retains C3b-inactivating and iC3b-degradation activity, and has at least 70%, 80%, 90%, 95%, 96%, 97%, 98%, or 99% amino acid sequence identity across its entire length to human Factor I with an amino acid sequence of SEQ ID NO: 2 or 4. The fragment or derivative may comprise one or more amino acid additions, deletions, or substitutions, for example one or more conservative amino acid substitutions.

Conservative amino acid substitutions are likely to have minimal impact on the activity of the resultant protein. Further information about conservative substitutions can be found, for instance, in Ben Bassat et al. (J. Bacterial., 169:751-757, 1987), O'Regan et al. (Gene, 77:237-251, 1989), Sahin-Toth et al. (Protein Sci., 3:240-247, 1994), Hochuli et al. (Bio/Technology, 6:1321-1325, 1988) and in widely used textbooks of genetics and molecular biology. The Blosum matrices are commonly used for determining the relatedness of polypeptide sequences. The Blosum matrices were created using a large database of trusted alignments (the BLOCKS database), in which pairwise sequence alignments related by less than some threshold percentage identity were counted (Henikoff et al., Proc. Natl. Acad. Sci. USA, 89:10 915-10 919, 1992). A threshold of 90% identity was used for the highly conserved target frequencies of the BLOSUM90 matrix. A threshold of 65% identity was used for the BLOSUM65 matrix. Scores of zero and above in the Blosum matrices are considered "conservative substitutions" at the percentage identity selected. The following table shows exemplary conservative amino acid substitutions:

| **Original Residue** | **Very Highly - Conserved Substitutions** | **Highly Conserved Substitutions (from the Blosum90 Matrix)** | **Conserved Substitutions (from the Blosum65 Matrix)** |
|---|---|---|---|
| Ala | Ser | Gly, Ser, Thr | Cys, Gly, Ser, Thr, Val |
| Arg | Lys | Gln, His, Lys | Asn, Gln, Glu, His, Lys |
| Asn | Gln; His | Asp, Gln, His, Lys, Ser, Thr | Arg, Asp, Gin, Glu, His, Lys, Ser, Thr |
| Asp | Glu | Asn, Glu | Asn, Gin, Glu, Ser |
| Cys | Ser | None | Ala |
| Gln | Asn | Arg, Asn, Glu, His, Lys, Met | Arg, Asn, Asp, Glu, His, Lys, Met, Ser |
| Glu | Asp | Asp, Gln, Lys | Arg, Asn, Asp, Gln, His, Lys, Ser |
| Gly | Pro | Ala | Ala, Ser |
| His | Asn; Gin | Arg, Asn, Gln, Tyr | Arg, Asn, Gln, Glu, Tyr |
| Ile | Leu; Val | Leu, Met, Val | Leu, Met, Phe, Val |
| Leu | Ile; Val | Ile, Met, Phe, Val | Ile, Met, Phe, Val |
| Lys | Arg; Gin; Glu | Arg, Asn, Gln, Glu | Arg, Asn, Gln, Glu, Ser, |
| Met | Leu; Ile | Gln, Ile, Leu, Val | Gln, Ile, Leu, Phe, Val |
| Phe | Met; Leu; Tyr | Leu, Trp, Tyr | Ile, Leu, Met, Trp, Tyr |
| Ser | Thr | Ala, Asn, Thr | Ala, Asn, Asp, Gln, Glu, Gly, Lys, Thr |
| Thr | Ser | Ala, Asn, Ser | Ala, Asn, Ser, Val |
| Trp | Tyr | Phe, Tyr | Phe, Tyr |
| Tyr | Trp; Phe | His, Phe, Trp | His, Phe, Trp |
| Val | Ile; Leu | Ile, Leu, Met | Ala, Ile, Leu, Met, Thr |

The fragment or derivative of Factor I may retain at least 50%, 60%, 70%, 80%, 90%, or 95%, or 100% of the C3b-inactivating and iC3b-degradation activity of native Factor I. The C3b-inactivating and iC3b-degradation activity of the fragment or derivative of Factor I, and native Factor I, may be determined using any suitable method known to those of skill in the art. For example, measurement of Factor I proteolytic activity is described in Hsiung et al. (Biochem. J. (1982) 203, 293-298), at page 295, left column. Both haemolytic and conglutinating assays for FI activity are described in Lachmann PJ & Hobart MJ (1978) "Complement Technology" in Handbook of Experimental Immunology 3rd edition Ed DM Weir Blackwells Scientific Publications Chapter 5A p17. A more detailed description, also including a proteolytic assay, is given by Harrison RA(1996) in "Weir's Handbook of Experimental Immunology" 5th Edition Eds; Herzenberg Leonore A 'Weir DM, Herzenberg Leonard A & Blackwell C Blackwells Scientific Publications Chapter 75 36-37. The conglutinating assay is highly sensitive and can be used for detecting both the first (double) clip converting fixed C3b to iC3b and acquiring reactivity with conglutinin; and for detecting the final clip to C3dg by starting with fixed iC3b and looking for the loss of reactivity with conglutinin. The haemolytic assay is used for the conversion of C3b to iC3b, and the proteolytic assay detects all the clips.

In some embodiments, the nucleic acid encoding Factor I encodes human Factor I with an amino acid sequence of SEQ ID NO: 2 (variant 2, with 18-residue N-terminal signal sequence, NCBI Reference Sequence: NM_000204.4), or SEQ ID NO: 4 (variant 2, without 18-residue N-terminal signal sequence).

In some embodiments, the nucleic acid encoding human Factor I comprises nucleotide sequence of SEQ ID NO: 1 (the coding region of the CDS for Factor I, transcript variant 2, mRNA, from NCBI Reference Sequence: NM_000204.4, with 18-residue N-terminal signal sequence), or SEQ ID NO: 3 (the coding region of the CDS for Factor I, transcript variant 2, mRNA, without 18-residue N-terminal signal sequence).

In some embodiments, a codon optimized nucleotide sequence encoding Factor I, or the fragment or derivative thereof, may be used. Suitable codon optimized sequences may be generated, for example, using codon optimization software, such as SnapGene.

An example of a codon optimized nucleotide sequence encoding Factor I is SEQ ID NO: 7. Thus, in some embodiments, the nucleic acid encoding Factor I comprises, or consists of, the nucleotide sequence of SEQ ID NO: 7.

In some embodiments, the nucleic acid encoding Factor I, or the fragment or derivative thereof, may comprise nucleic acid that has at least 10%, 20%, 30%, 40%, 50%, 60%, 70%, 80%, 90%, 95%, 96%, 97%, 98%, or 99% nucleic acid sequence identity across its entire length to nucleic acid encoding human Factor I with a nucleotide sequence of SEQ ID NO: 1 or 3.

"Percent (%) sequence identity" with respect to a reference polypeptide or nucleic acid sequence is defined herein as the percentage of amino acid residues or nucleotides in a sequence that are identical with the amino acid residues or nucleotides in the reference polypeptide or nucleic acid sequence, after aligning the sequences and introducing gaps, if necessary, to achieve the maximum percent sequence identity. Alignment for purposes of determining percent amino acid or nucleic acid sequence identity can be achieved in various ways that are within the skill in the art, for example, using publicly available computer software programs, such as those described in Current Protocols in Molecular Biology (Ausubel et al., eds., 1987), Supp. 30, section 7.7.18, Table 7.7.1, and including BLAST, BLAST-2, ALIGN or Megalign (DNASTAR) software. An example of an alignment program is ALIGN Plus (Scientific and Educational Software, Pennsylvania). Those skilled in the art can determine appropriate parameters for measuring alignment, including any algorithms needed to achieve maximal alignment over the full length of the sequences being compared. For purposes herein, the percentage amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y, where X is the number of amino acid residues scored as identical matches by the sequence alignment program in that program's alignment of A and B, and where Y is the total number of amino acid residues in B. It will be appreciated that where the length of amino acid sequence A is not equal to the length of amino acid sequence B, the percentage amino acid sequence identity of A to B will not equal the percentage amino acid sequence identity of B to A. For purposes herein, the percentage nucleic acid sequence identity of a given nucleic acid sequence C to, with, or against a given nucleic acid sequence D (which can alternatively be phrased as a given nucleic acid sequence C that has or comprises a certain percentage nucleic acid sequence identity to, with, or against a given nucleic acid sequence D) is calculated as follows: 100 times the fraction W/Z, where W is the number of nucleotides scored as identical matches by the sequence alignment program in that program's alignment of C and D, and where Z is the total number of nucleotides in D. It will be appreciated that where the length of nucleic acid sequence C is not equal to the length of nucleic acid sequence D, the percentage nucleic acid sequence identity of C to D will not equal the percentage nucleic acid sequence identity of D to C.

The subject referred to herein is a mammal. Suitable examples include domesticated animals (such as cows, sheep, cats, dogs, horses), primates (e.g., humans and non-human primates such as monkeys), rabbits, and rodents (e.g., mice and rats). In certain embodiments, the subject is a human.

As used herein, "treat" or "treatment" is an approach for obtaining beneficial or desired clinical results For purposes of this invention, beneficial or desired clinical results include alleviation of symptoms, diminishment of extent of disease, stabilized (e.g., not worsening) state of disease, preventing spread of disease, delay or slowing of disease progression, amelioration or palliation of the disease state, reduction in the required dose and/or frequency of administration of a pre-existing treatment regimen, and remission (whether partial or total), whether detectable or undetectable. "Treatment" can also mean prolonging survival as compared to expected survival if not receiving treatment. Treatment may be applied for a sufficient period of time and/or at a sufficient frequency to obtain the beneficial or desired clinical results.

As used herein, "prevent" or "prevention" refers to treatment, wherein an individual is known or suspected to have or be at risk for having a disorder but has displayed no symptoms or minimal symptoms of the disorder. An individual undergoing preventative treatment may be treated prior to onset of symptoms.

A "therapeutically effective amount" is an amount sufficient to effect beneficial or desired results, including clinical results (for example, amelioration of symptoms, achievement of clinical endpoints). A therapeutically effective amount can be administered in one or more administrations. In terms of a disease state, a therapeutically effective amount is an amount sufficient to ameliorate, stabilize, or delay development of a disease.

It will be appreciated that a recombinant viral vector of the invention will generally be an isolated recombinant viral vector, i.e. separated and/or recovered from a component of its natural environment. Similarly, a recombinant virus particle of the invention will generally be an isolated recombinant virus particle, i.e. separated and/or recovered from a component of its natural environment.

There is also provided according to the invention a pharmaceutical composition, which comprises: a recombinant viral vector of the invention, or a recombinant virus particle of the invention, and a pharmaceutically acceptable carrier, excipient, or diluent.

A pharmaceutical composition of the invention may be suitable for any appropriate mode of administration of the composition, for example intravenous administration, or any further appropriate mode of administration described above.

Pharmaceutical compositions of the invention may be suitable for administration to a human subject.

There is also provided according to the invention a kit, which comprises: a recombinant viral vector of the invention, or a recombinant virus particle of the invention, and a pharmaceutically acceptable carrier, excipient, or diluent.

In some embodiments, a kit of the invention may further comprise instructions for treating a complement-mediated disorder described herein using any of the methods, vectors, or virus particles of the invention. A kit of the invention may include a pharmaceutically acceptable carrier, excipient, or diluent suitable for injection of a subject in need of treatment. A kit of the invention may further comprise any of the following: a buffer, a filter, a needle, a syringe, and a package insert with instructions for performing administration into the subject. Suitable packaging materials may also be included and may be any packaging materials known in the art, including, for example, vials (such as sealed vials), vessels, ampules, bottles, jars, flexible packaging (e.g., sealed Mylar or plastic bags). These packaging materials may further be sterilized and/or sealed.

A pharmaceutical composition, or a kit of the invention may be packaged in single unit dosages or in multi-dosage forms. A pharmaceutical composition of the invention, and the contents of a kit of the invention, are generally formulated as sterile and substantially isotonic solution.

Pharmaceutically acceptable carriers, excipients, and diluents are relatively inert substances that facilitate administration of a pharmacologically effective substance and can be supplied as liquid solutions or suspensions, as emulsions, or as solid forms suitable for dissolution or suspension in liquid prior to use. For example, an excipient can give form or consistency, or act as a diluent. Suitable excipients include but are not limited to stabilizing agents, wetting and emulsifying agents, salts for varying osmolarity, encapsulating agents, pH buffering substances, and buffers. Such excipients include any pharmaceutical agent suitable for direct delivery to the subject (for example intravenously, or to the liver) which may be administered without undue toxicity. Pharmaceutically acceptable excipients include, but are not limited to, sorbitol, any of the various TWEEN compounds, and liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, or benzoates.

In some embodiments, pharmaceutically acceptable excipients may include pharmaceutically acceptable carriers. Such pharmaceutically acceptable carriers can be sterile liquids, such as water and oil, including those of petroleum, animal, vegetable or synthetic origin, such as peanut oil, soybean oil, mineral oil. Saline solutions and aqueous dextrose, polyethylene glycol (PEG) and glycerol solutions can also be employed as liquid carriers, particularly for injectable solutions. Additional ingredients may also be used, for example preservatives, buffers, tonicity agents, antioxidants and stabilizers, non-ionic wetting or clarifying agents, or viscosity-increasing agents.

A thorough discussion of pharmaceutically acceptable excipients and carriers is available in Remington's Pharmaceutical Sciences (Mack Pub. Co., N.J. 1991).

In some embodiments, the viral titre of a pharmaceutical composition of the invention is at least 5 x 10¹², 6 x 10¹², 7x 10¹², 8 x 10¹², 9 x 10¹², 10 x 10¹², 11 x 10¹², 15 x 10¹², 20 x 10¹², 25 x 10¹², 30 x 10¹², or 50 x 10¹² genome copies/mL. In some embodiments, the viral titre of the composition is 5 x 10¹² to 10 x 10¹², 10 x 10¹² to 25 x 10¹², or 25 x 10¹² to 50 x 10¹² genome copies/mL. In some embodiments, the viral titre of the composition is 5 x 10¹² to 6 x 10¹², 6 x 10¹² to 7 x 10¹², 7 x 10¹² to 8 x 10¹², 8 x 10¹² to 9 x 10¹², 9 x 10¹² to 10 x 10¹², 10 x 10¹² to 11 x 10¹², 11 x 10¹² to 15 x 10¹², 15 x 10¹² to 20 x 10¹², 20 x 10¹² to 25 x 10¹², 25 x 10¹² to 30 x 10¹², 30 x 10¹² to 50 x 10¹², or 50 x 10¹² to 100 x 10¹² genome copies/mL.

In some embodiments, the viral titre of a pharmaceutical composition of the invention is at least 5 x 10⁹, 6 x 10⁹, 7 x 10⁹, 8 x 10⁹, 9 x 10⁹, 10 x 10⁹, 11 x 10⁹, 15 x 10⁹, 20 x 10⁹, 25 x 10⁹, 30 x 10⁹, or 50 x 10⁹ transducing units /mL. In some embodiments, the viral titre of the composition is 5 x 10⁹ to 10 x 10⁹, 10 x 10⁹ to 15 x 10⁹, 15 x 10⁹ to 25 x 10⁹, or 25 x 10⁹ to 50 x 10⁹ transducing units /mL. In some embodiments, the viral titre of the composition is 5 x 10⁹ to 6 x 10⁹, 6 x 10¹ to 7 x 10⁹, 7 x 10⁹ to 8 x 10⁹, 8 x 10⁹ to 9 x 10⁹, 9 x 10⁹ to 10 x 10⁹, 10 x 10⁹ to 11 x 10⁹, 11 x 10⁹ to 15 x 10⁹, 15 x 10⁹ to 20 x 10⁹, 20 x 10⁹ to 25 x 10⁹, 25 x 10⁹ to 30 x 10⁹, 30 x 10⁹ to 50 x 10⁹ or 50 x 10⁹ to 100 x 10⁹ transducing units/mL.

The term "transducing unit" as used in reference to a viral titre, refers to the number of infectious recombinant vector particles that result in the production of a functional transgene product as measured in functional assays, such as described in the Examples in WO 2015/168666, or for example, in Xiao et al. (1997) Exp. Neurobiol., 144:113-124; or in Fisher et al. (1996) J. Virol., 70:520-532 (LFU assay).

In some embodiments, the viral titre of a pharmaceutical composition of the invention is at least 5 x 10¹⁰, 6 x 10¹⁰, 7 x 10¹⁰, 8 x 10¹⁰, 9 x 10¹⁰, 10 x 10¹⁰, 11 x 10¹⁰, 15 x 10¹⁰, 20 x 10¹⁰, 25 x 10¹⁰, 30 x 10¹⁰, 40 x 10¹⁰, or 50 x 10¹⁰ infectious units/mL. In some embodiments, the viral titre of the composition is at least 5 x 10¹⁰ to 10 x 10¹⁰, 10 x 10¹⁰ to 15 x 10¹⁰, 15 x 10¹⁰ to 25 x 10¹⁰, or 25 x 10¹⁰ to 50 x 10¹⁰ infectious units/mL. In some embodiments, the viral titre of the composition is at least 5 x 10¹⁰ to 6 x 10¹⁰, 6 x 10¹⁰ to 7 x 10¹⁰, 7 x 10¹⁰ to 8 x 10¹⁰, 8 x 10¹⁰ to 9 x 10¹⁰, 9x 10¹⁰ to 10 x 10¹⁰, 10 x 10¹⁰ to 11 x 10¹⁰, 11 x 10¹⁰ to 15 x 10¹⁰, 15 x 10¹⁰ to 20 x 10¹⁰, 20 x 10¹⁰ to 25 x 10¹⁰, 25 x 10¹⁰ to 30 x 10¹⁰, 30 x 10¹⁰ to 40 x 10¹⁰, 40 x 10¹⁰ to 50 x 10¹⁰, or 50 x 10¹⁰ to 100 x 10¹⁰ infectious units/mL.

The term "infectious unit" as used in reference to a viral titre, refers to the number of infectious and replication-competent recombinant vector particles as measured by the infectious centre assay, also known as replication centre assay, as described, for example, in McLaughlin et al. (1988) J. Virol., 62:1963-1973.

There is further provided according to the invention a kit for production of rAAV particles, which comprises: a rAAV vector of the invention, and one or more helper plasmids comprising nucleic acid encoding AAV replication and capsid proteins, and genes required for a productive AAV life cycle.

In certain embodiments, a kit of the invention for production of rAAV particles comprises a first helper plasmid comprising the nucleic acid encoding AAV replication and capsid proteins, and a second helper plasmid comprising the nucleic acid encoding genes required for a productive AAV life cycle.

The genes required for a productive AAV life cycle may be adenoviral, herpesvirus, or baculovirus genes. In particular embodiments, the genes are adenoviral genes, in particular E1a, E1b, E2a, E4 and VA RNA adenoviral genes.

There is further provided according to the invention a recombinant viral vector of the invention, a recombinant virus particle of the invention, or a pharmaceutical composition of the invention, for use as a medicament.

The invention also provides a recombinant viral vector of the invention, a recombinant virus particle of the invention, or a pharmaceutical composition of the invention, for use in the treatment of a complement-mediated disorder.

There is also provided according to the invention use of a recombinant viral vector of the invention, a recombinant virus particle of the invention, or a pharmaceutical composition of the invention, in the manufacture of a medicament for the treatment of a complement-mediated disorder.

The complement-mediated disorder may be a disorder associated with a defect in alternative pathway regulation, and in particular with over-activity of the complement C3b feedback cycle.

Examples of complement-mediated disorders that may be prevented or treated according to the invention include age-related macular degeneration (AMD) (particularly early (dry) AMD, or geographic atrophy), dense deposit disease (DDD), atypical haemolytic uraemic syndrome (aHUS), C3 glomerulopathies, membranoproliferative glomerulonephritis Type 2 (MPGN2), atherosclerosis, chronic cardiovascular disease, Alzheimer's disease, paroxysmal nocturnal haemoglobinuria (PNH), autoinflammatory diseases of old age.

Further examples of complement-mediated disorders that may be prevented or treated according to the invention include MPGN type I, MPGN type III, Guillain-Barré syndrome, Henoch-Schonlein purpura, IgA nephropathy, and membranous glomerulonephritis.

Embodiments of the invention are now described, by way of example only, with reference to the accompanying drawings in which:
Figure 1 shows an outline of the steps of complement activation;
Figure 2 shows the feedback loop of the alternative pathway of vertebrate complement;
Figure 3 shows the proteolysis of C3 during complement activation. C3 consists of the α and β chain. The β-chain is not modified while the a-chain is cleaved several times: i) C3a is cleaved off by a C3 convertase and the remainder protein is now called C3b; ii) FI cleavage at two sites, releasing the C3f peptide (FH as co-factor), it is now called iC3b or C3bi; iii) with CR1 as co-factor, FI cleaves again in the 68 kDa fragment of iC3b. While C3c diffuses off. C3dg stays attached to the cell surface and can be cleaved further by trypsin-like proteases;
Figure 4 shows a schematic representation of AAV gene therapy;

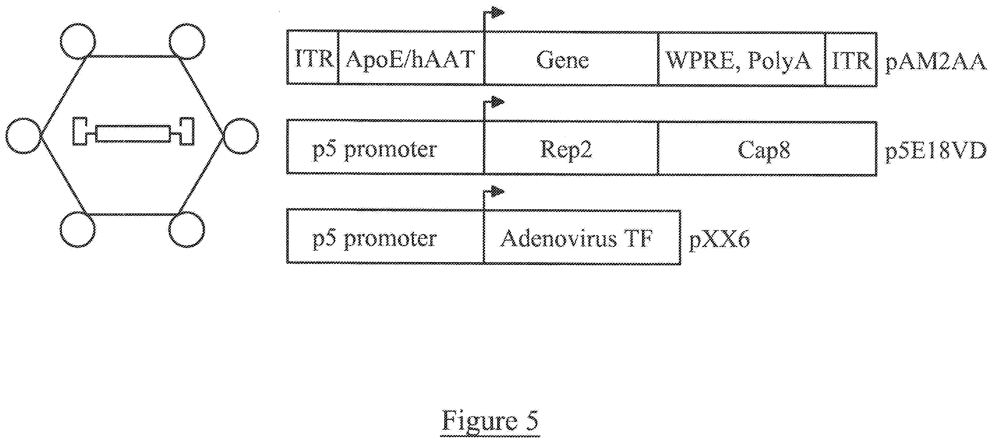

Figure 5 shows an AAV2/8 construct used for over expression of serum levels of Factor I. The transgene, here Factor I, is inserted between a promoter and a polyadenylation recognition site. Flanking on both sides are two inverted terminal repeats (ITR). The capsid sequence and further adenoviral genes are packed into other plasmids;
Figure 6 shows elevated Factor I levels measured by western blot. Mouse serum was diluted to 5% and 10µl were separated by SDS PAGE. FI was detected with an α-FI antibody (1:500) that reacts with the heavy chain of mFl. The experiment was performed twice;

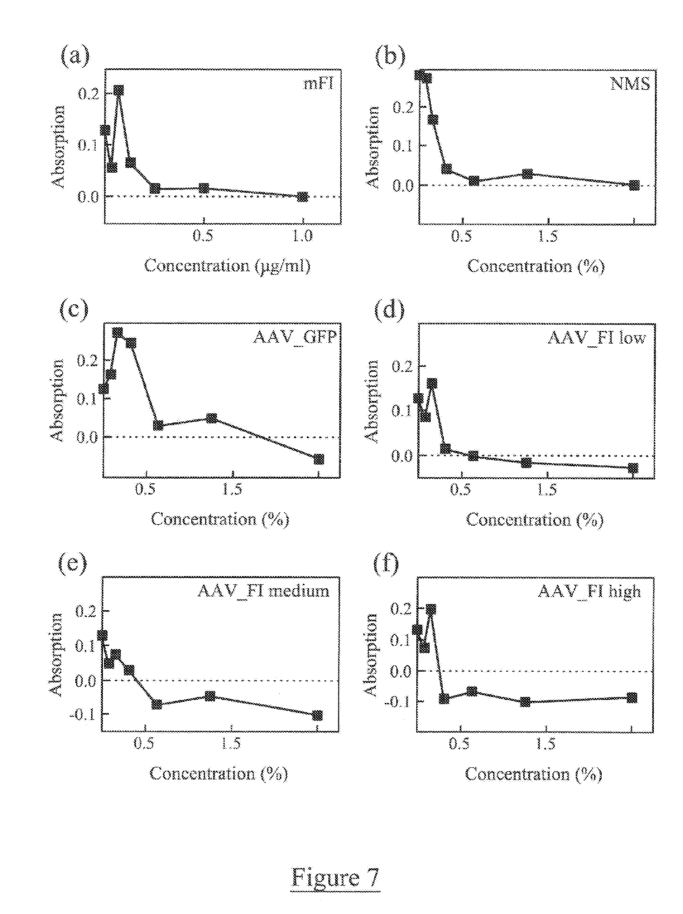

Figure 7 shows representative results from an inhibition ELISA. (a) The assay is calibrated with known concentrations of purified recombinant mFI. Concentrations lower than 0.25µg/ml give a positive signal. (b) Normal mouse serum (NMS) is positive at concentrations lower than 0.625%. (c-f) Inhibition ELISA using sera from mice injected with AAV-constructs. Only one serum sample per group is shown here. The experiment was performed twice in triplicate;

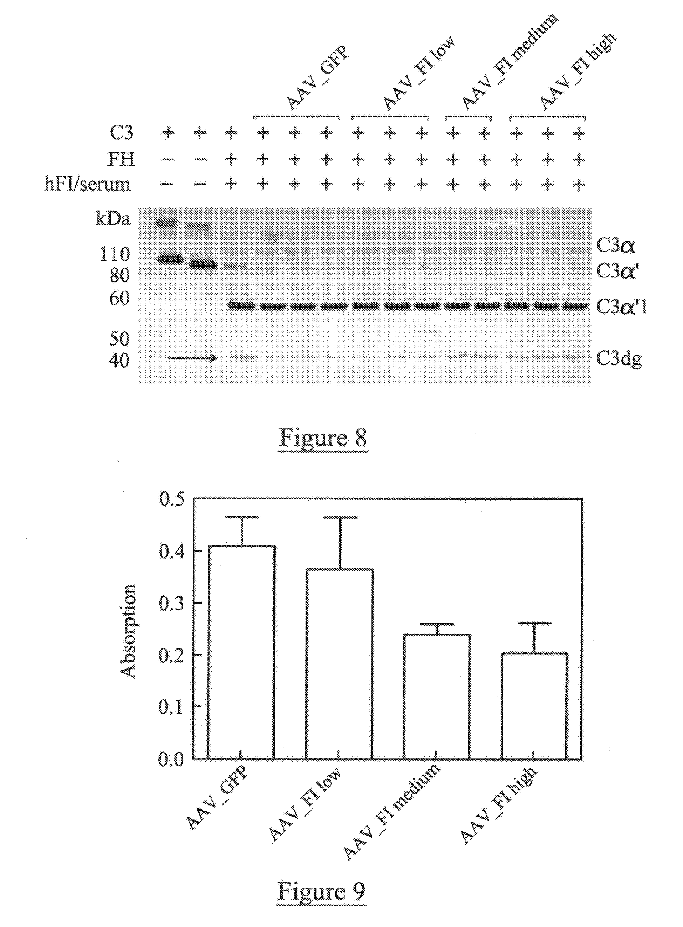

Figure 8 shows the results of a C3b and iC3b *in vitro* cleavage assay to measure functional activity of over-expressed Factor I. Substrate, i.e. C3α'1 chain, is generated quickly and the rate of its breakdown is compared, i.e. generation of C3dg. C3 cleavage was detected with α-hC3dg (1µg/ml). The experiment was performed twice; and
Figure 9 shows the results of an iC3b deposition assay to test functional activity of over-expressed Factor I. Serum of injected mice was diluted to 25% in alternative pathway complement fixation buffer and loaded onto an LPS-coated plate. After incubation for 1 hour at 37°C, bound iC3b was detected with an α-human C3c antibody. Absorption was measured at 415nm. The experiment was performed twice in duplicate.

### Example 1

### AAV-expression system

This example describes preparation of a recombinant viral vector encoding murine Factor I that enables over-expression of Factor I in murine hepatocytes, and preparation of viral particles (virions) encapsidating the vector.

An adeno-associated virus (AAV) construct was used. It consists of an AAV2 viral backbone that was pseudotyped with the AAV8 capsid protein in order to confer liver tropism. The virus, therefore, mainly infects the liver, and the FI transgene is over-expressed at its natural site. To further suppress extra-hepatic expression of Factor I transgene, an α-1-anti-trypsin (AAT) promoter with two additional ApoE hepatic control regions was used (see Figure 5). All required genes for virus production are split up between three plasmids that are co-transfected into a cell line that provides the remaining missing genes for AAV packaging, as further described below.

AAV (wild type): The 4.7kb genome of wildtype AAV is characterized by two inverted terminal repeats (ITRs) and two sets of open reading frames (ORFs), which encode the replication (Rep) and capsid (Cap) proteins. The Rep ORFs encode four proteins (78, 68, 52, and 40 kDa), which function mainly in regulating AAV replication and integration. The Cap ORFs encode three structural proteins (85kDa (VP1), 72kDa (VP2) and 61kDa (VP3)). VP1:VP2:VP3 ratios are approximately 1:1:8 or 1:1:10 in the capsid.

Recombinant AAV (rAAV): rAAV used in this example is AAV2 pseudotyped with AAV8 capsid (rAAV2/8) to confer liver tropism. The rAAV2/8 virions are packaged in HEK293 cells by triple transfection using the three plasmids described below:
1. pAM2AA (ITRs and transgene expression cassette): In this plasmid the 145 bp inverted terminal repeats (ITRs) from AAV2 flank the transgene cassette. The two ITRs are the only cis elements essential for all steps in the AAV life cycle. They function as the origin of DNA replication, provide packaging and integration signals, and serve as regulatory elements for WT AAV gene expression. The pAM2AA cassette includes the human α-1-antitrypsin (hAAT) promoter with two ApoE enhancers followed by the cDNA encoding the transgene. The 3'-untranslated region (UTR) contains the woodchuck hepatitis post-transcriptional regulatory element (WPRE) and bovine growth hormone polyadenylation signal (BGH polyA). The packaging capacity of WT AAV is approximately 4.7 kb. In pAM2AA, the regulatory regions take up = 2390 bp, leaving ≈ 2310bp for insertion transgenes.
2. p5E18VD/8 (AAV helper sequences): The deleted viral coding sequences from AAV2, including Rep and Cap genes are present in this plasmid driven by the p5 AAV promoter. Here, the sequences encoding AAV2 capsid are replaced by sequences encoding AAV8 capsid. There is no homology between vector and helper sequences, reducing the possibility of generating wild type AAV recombinants.
3. pXX6 (adenoviral helper functions): The adenoviral genes essential for a productive AAV life cycle are the E1a, E1b, E2a, E4 and VA RNA genes. E1a serves as a transactivator which upregulates transcription of adenoviral genes as well as the AAV Rep and Cap genes. E1b interacts with E4 to facilitate transportation of viral mRNAs. E4 is also involved in facilitating AAV DNA replication. E2a and VA RNA act to enhance the viral mRNA stability and efficiency of translation especially for the Cap transcripts. pXX6 contains the essential adenoviral helper genes but lacks adenovirus structural and replication genes. Essential helper genes included in pXX6 are E2a, E4 and VA genes. Both E1a and E1b have been deleted, and the missing E1a and E1b genes are complemented by HEK293 cells.

### Preparations of recombinant AAV vector encoding murine Factor I (AAV mFI)

To enable cloning of Factor I into the pAM2AA expression vector, the restriction sites had to be changed into compatible enzyme recognition sites (using primer sequences: FI-AAV-F: 5'- TCT AGA GGA TCC GCC ACC ATG AAG CTC - 3' (SEQ ID NO: 5); and FI-AAV-R: 5'-AAG CTT GGC GGC CGC TCA GAC ATT GTG TTG AGA AAC AAG AGA CCT TC - 3'(SEQ ID NO:6). The new sequences were attached to the mouse Factor I cDNA via PCR amplification. After purification of the amplified construct, it was ligated into pAM2AA. The cDNA of murine complement Factor I was cloned into pAM2AA using Xbal and Hindlll restriction sites. Once the vector had been prepared, an AAV_GFP vector was treated the same way alongside all subsequent virus packaging and purification steps. This ensures that no errors occurred since expression of hepatic GFP can easily be tracked visually under a fluorescence microscope.

### Preparation of rAAV2/8 virions

The pAM2AA vector with cDNA of murine complement Factor I was transformed into SURE2 cells to prevent unwanted recombination events. HEK293 cells were transfected with all three plasmids using CaPO₄. Two days after transfection, cells were harvested and cells were lysed by repeated freeze-thawing in a dry ice/100% ethanol bath and then stored at -80°C. AAV virions were purified on a cesium chloride gradient and virus particles were quantified by real-time PCR.

### Example 2

### In vivo overexptossion of complement Factor I by an adeno-associated virus delivery system

This example describes injection of mice with virions encapsidating the AAV_mFI vector produced as described in Example 1, determination of plasma levels of Factor I following injection of the virions, and an assessment of the down-regulation of the alternative complement pathway in the mice following injection.

### Injection of mice

12 mice were divided into 4 groups, which received different concentrations of AAV_mFI or the control virus preparation:
- low mFI = 5x10⁹ virions
- med mFI = 5x10¹⁰ virions (=100 % liver transduction in previous experiments)
- high mFl = 5x10¹¹ virions
- GFP = 5x10¹¹ virions

The mice were injected intravenously into the tail vein. Blood was taken after 4 weeks, and after 8 weeks the mice were culled and their serum was analysed.

### Results

### Quantification of elevated Factor I levels

To quantify FI levels, a polyclonal custom-made α-mFI antiserum was ordered. In the meantime, a western blot was performed. Here, α-FI (Santa Cruz, sc-69465) was used as detecting antibody to roughly quantify FI levels. Serum was diluted in sequential steps to reduce pipetting errors. The relative concentration of FI was determined as follows: AAV_GFP < AAV_FI low < AAV_FI medium < AAV_FI high (see Figure 6).

Once the antiserum arrived, a much more sensitive assay, an inhibition ELISA, was performed. Serum was pre-incubated with a before-determined concentration of α-mFI-antiserum and then incubated on a microtiter plate coated with recombinant mFl. Preparation and binding specificity of the α-mFI-antiserum are described in the Materials and Methods section below. Only unbound antibodies are able to react with the immobilised antigen on the plate. Therefore, the assay only gives a positive result if the amount of α-mFI-antibodies in the antiserum exceeds the mFI present in serum. The assay is calibrated with known Factor I concentrations.

It was found that antiserum concentrations above 75 µg/ml give a strong positive signal when the microtiter plate is coated with 1µg mFI per well (not shown). Figure 7 shows representative results from an inhibition ELISA. The assay gives a positive signal at concentrations lower than 0.25µg/ml (Figure 7a). Using this calibration, the concentration of FI in a serum sample can be determined. First, a normal sample of wild-type mouse was tested and concentrations lower than 0.625% gave a positive signal (Figure 7b). Knowing that the threshold of a positive signal in this assay is 0.25µg/ml mFl, the concentration of mFI in 100% serum can be calculated, i.e. 40 µg/ml. The same calculation was done for each of the mouse sera injected with the AAV-constructs (Figure 7c-f), and the concentration range per group was determined (see Table 2).

**Table 2. Quantification of Factor I after over-expression by an adeno-associated virus expression system**

| **Group** | **FI *µ*g/mL** | **FI increase** |
|---|---|---|
| AAV_GFP | 20-40 | 1x |
| AAV_FI low | 40-80 | 1-2x |
| AAV_FI medium | 80 | 2x |
| AAV_FI high | 80-160 | 2-4x |

Starting from 20-40µg/ml FI in the GFP control group, the concentration is 40-80µg/ml in the low-, 80µg/ml in the medium- and 80-160µg/ml FI in the high FI dose. It was concluded that the serum concentration can be raised up to four times normal levels by AAV gene therapy.

### Functional analysis of serum with elevated Factor I levels

The serum of the transgenic mice was analysed in a similar manner as the recombinant Factor I. Since the over-expressed protein will, theoretically, be processed and secreted as endogenous FI, the sera of the mice could be directly compared with each other. After FI increase has been confirmed by immunoblotting and by an inhibition ELISA, the functional activity of the over-expressed enzyme had to be confirmed. Therefore, the sera were analysed for FI functional activity by an *in vitro* C3b cleavage assay, a hemolysis assay and an iC3b deposition assay.

C3b and iC3b cleavage was measured in an *in vitro* assay. Human C3 was digested to C3b with trypsin and incubated with hFH and serum from transgenic mice. After 30 minutes, 1 µg human C3 was loaded onto a SDS gel and blotted with clone 9. C3b is first cleaved into iC3b in a quick reaction (the antibody reacts with the C3α'1 chain of iC3b) and then further into C3dg in a much slower reaction. This second reaction can be speeded up by increased FI concentrations. It is shown that human C3b incubated with human Factor H and serum from the different mouse groups was more efficiently cleaved to C3dg within the incubation period in the AAV_FI medium and AAV_FI high group (see Figure 8, lanes 10-14). Mice that have been injected only with a control plasmid show almost no C3dg band within the incubation period.

The next step was to show that over-expressed Factor I was also functionally active in whole serum. For this, a microtiter plate was coated with LPS and C3b/iC3b deposition was measured after incubation with transgenic mouse serum at 37°C. The results are shown in Figure 9. As with the recombinant protein, mice injected with AAV_mFI showed less C3b/iC3b deposition on LPS since their positive feedback loop of the alternative pathway was interrupted by Factor I. C3b/iC3b deposition was reduced by 11% in the AAV_low FI, by 38% in the AAV_medium FI and by 50% in the AAV% high FI group.

It was concluded that complement Factor I can be over-expressed by gene therapy, using a viral vector, to levels which cause significant down-regulation of the alternative complement pathway.

### Discussion

Gene therapy is a new and exciting therapeutic option for the treatment of various diseases. It enables *in vivo* expression of proteins after transfection of the respective cell with a construct harbouring the DNA sequence of the protein of interest. Theoretically, gene therapy can be utilised for expression of every protein, although practically, there are usually limitations, such as for example the length of the sequence. Using gene therapy, missing proteins can be replaced. However, this always carries a risk of immunogenicity. We have appreciated that this risk does not occur if concentrations of existing (endogenous) proteins are raised. In this study we wanted to elucidate whether complement Factor I can be over-expressed by gene therapy to levels which cause significant down-regulation of the alternative complement pathway.

We have succeeded in generating a vector for transgenic expression of mouse complement Factor I. The viral expression system used is based on AAV2 and its capsid is derived from AAV8. Together this AAV2/8 construct mainly results in liver transduction in mice, although virus particles can be also found in extra-hepatic tissue. To increase liver-specific expression, the transgene is under the control of the α-1-anti-trypsin promoter with two ApoE hepatic control regions for high-level and specific expression in hepatocytes. Therefore, transgene expression should only occur in hepatocytes (although, sometimes there can be very little transgene expression also in pancreatic tissue, which is a very closely related tissue).

We have demonstrated that over-expression of FI by the AAV expression system used clearly has an effect on complement down-regulation and secondly that this effect is titrate-able and becomes more profound as the vector dose is increased. In order to measure the total FI increase, a polyclonal antibody against mouse Factor I was raised in a rabbit, since commercially available α-FI antibodies were found unsuitable for measuring mouse FI concentrations in an ELISA (they do not react with native FI but only with the denatured protein in a western blot). Using this polyclonal custom-made antibody it was possible to show that serum concentrations were increased up to four times normal levels, i.e. from 20-40µg/ml in control mice to 80-160µg/ml FI in transgenic mice.

Another interesting question is whether transgene expression is further increased in an acute phase reaction. Since in the construct used, FI is under the control of the α-1-anti- trypsin promoter (α-1-anti-trypsin and FI are both positive acute phase reactants), it can be expected that transgene expression will also be increased in an acute phase reaction. This could be tested in a straightforward animal experiment that could be easily performed by injection of LPS or IL6 into wildtype and transgenic mice and then compare the rate of FI increase. Assuming both endogenous and transgenic FI expression would increase, then this could be a solution to reduce the initial virus load during AAV administration but with the option to increase expression if required.

Factors such as host immunity have prevented the widespread use of AAV in man. Once exposed to AAV, people develop neutralizing antibodies that block gene delivery. However, by engineering of the capsid proteins, new variations can be generated that have higher transduction efficiencies and are not recognised by neutralising antibodies. New approaches to limit these immune responses are therefore undertaken (Mingozzi, et al., Science Translational Medicine, 5(194):194ra92-194ra92, July 2013). Recent clinical studies have shown success of a single infusion of an AAV vector leading to two years of therapeutic levels of Factor IX in men with severe hemophilia B (Mingozzi and High, Blood, 122(1):23-36, July 2013), although it should be pointed out that an increase of only 1% of normal FIX levels, substantially ameliorates the severe bleeding phenotype in hemophilia B patients (Nathwani, et al., N. Engl. J. Med., 371(21):1994-2004, November 2014). Before, AAV transgene expression of FIX in skeletal muscle was shown to persist for 10 years, although in this case, circulating FIX levels remained subtherapeutic (< 1%) (Buchlis, et al., Blood, 119(13):3038-3041, March 2012).

An increase of FI by 50% maximal is what is aimed for in human therapy. This is by far exceeded in mice using the AAV_mFI-construct, and the iC3b deposition assay shows a reduction of up to 50% less C3b deposition at the high AAV_mFI dose. Separately, we have shown that 50% more FI converts the activity of an high-risk complotype to the activity of a low-risk complotype (Lay *et al.,* 2015 (*supra*))*.* Since both of these complotypes are extremely rare and the majority of people will be within both extremes, an effect for risk reduction for the majority of people is only required within the range of 50% FI increase.

### Materials and Methods

### α-mouse Factor I

This polyclonal antibody is commercially available from Santa Cruz Biotechnology, Inc (# sc-69465). The antibody was raised in goats against a peptide in the heavy chain of mouse Factor I and recognizes the heavy chain in reducing and non-reducing western blots or ELISAs. If still intact, the antibody also recognises the pro-enzyme. Nevertheless, the antibody is not precipitating because the peptide used for immunisation of is too short.

Normal working concentration 1:500.

### α-mouse Factor I antiserum

This antibody is not commercially available and was ordered from Absea Biotechnology Ltd. in order to get a precipitating antibody to mFI which would allow easy determination of Factor I levels in a Ouchterlony double immunodiffusion assay. A rabbit was immunised with a peptide fragment (203aa-510aa) of recombinant mouse Factor I that was produced in bacteria. Once tested, the antibody turned out to be non-precipitating and therefore, an inhibition ELISA was developed to measure the levels of Factor I in serum. To get a multivalent antibody to mouse Factor I that precipitates FI, Factor I purified from mammalian cell culture was sent to Absea Biotechnology Ltd. but the immunisation period (8 weeks) for this second α-mouse Factor I antiserum was not completed in time but can be used for future experiments.

### Clone 9 antibody

Clone 9 is a rat α-human C3g antibody that recognises a neo-epitope in C3g that only becomes accessible if C3 is cleaved to iC3b or C3dg by Factor I (Lachmann, et al., Immunology, 41(3):503-515, November 1980). Under native conditions, clone 9 only reacts with iC3b or C3dg of human origin, whereas under denaturing conditions it detects the α, α' chain and the 68kDa fragment of human C3 because all these three fragments include its epitope in C3g. Normal working concentration for detection in a western blot is 0.5µg/ml and as capture antibody for coating is 1.35µg/ml. The names clone 9 and alpha-hC3g antibody will be used interchangeably.

### Clone 4 antibody

Clone 4 is a rat monoclonal anti-C3c antibody that recognizes a conformational epitope in C3c and reacts with C3, C3b, iC3b and C3c (Lachmann, et al., Immunology, 41(3):503-515, November 1980). It therefore does not bind to Cdg or C3g because of the absence of the epitope in C3c. Normal working concentration is 5 µg/ml.

### α-human C3c

This antibody is commercially available from Dako and is used to detect C3b and iC3b deposition. It is used at a concentration of 1:5000 and detected with an α-rabbit secondary antibody.

### Enzyme-linked immunosorbent assay (ELISA)

iC3b deposition assay - "clone 9 assay" Maxisorb Nunc Plates were coated overnight at 4°C with clone 9 at a concentration of 1.25µg/well in coating buffer. Next day, the plates were washed 5x with washing buffer (=PBS-0.05% Tween 20) and blocked with 4% Marvel milk powder in PBS-T for 2 hours at room temperature. The plates were washed 5x and diluted serum samples (1:2000 in PBS/gelatin) were added to each well (preparation of serum samples is described below). After a 1 hour incubation, the plate was washed and incubated for 1 hour with biotinylated clone 4 at a concentration of 5 µ g/ml in PBS/gelatin. The plates were washed 5x and incubated with 1:1000 Extravidin-HRP in PBS/gelatin. Finally, after extensive washing, 100µl TMB substrate (Invitrogen) were added and plates were incubated on a plate shaker for 30 minutes. The reaction was stopped with 50µl H₂SO₄ and the plates were read on a Microplate reader (450nm).

C3b deposition assay Maxisorb Nunc Plates were coated overnight with mannan (Sigma, mannan from *Saccharomyces cerevisiae*) or lipopolysaccharide (Sigma) 1µg/well at 4°C in coating buffer. On the next day, the plates were washed in TBS-0.05% Tween-20 and blocked with 1%BSA in TBS-T at room temperature. After 2 hours, the plates were washed and incubated with serial dilutions of recombinant proteins and/or sera. After a 1 hour incubation at 37°C, plates were washed with TBS-T and incubated for 1 hour with polyclonal rabbit α-human C3c complement (Dako), used 1:5000 in TBS-T. After extensive washing, α-rabbit IgG (whole molecule)-alkaline phosphatase (produced in goat, Sigma) 1:5000 in TBS-T was added for 1 hour. The plates were washed 4x with TBS-T and the assay was developed using Fast p-Nitrophenyl Phosphate Tablets (Sigma) and measured (405nm). Alternatively, the assay development was stopped by addition of 3 M NaOH and read afterwards.

Inhibition ELISA In an inhibition ELISA, microtiter plates are coated with antigen and, after blocking, the concentration of antiserum is determined that gives a strong signal when detected with the secondary antibody. This concentration is then used in the inhibition assay and mixed with serial dilutions of a sample with an unknown antigen concentration. At the same time, a dilution series of a known antigen concentration is prepared. During this preincubation step, immune complexes form and when the mix is loaded on the coated microtiter plate, only samples in which the concentration of antigen does not exceed the amount of specific antibody give a positive signal. If there is excess antigen, no free antibodies are available that can bind to the immobilised antigen on the microtiter plate. Therefore, a positive result in an inhibition assay shows the dilution of sample in which the amount of free antigen is limiting and by comparison with the standard dilutions of known antigen concentrations, the unknown concentration can be determined.

For determination of the concentration of mouse Factor I in a serum sample, Maxisorb Nunc Plates were coated overnight at 4°C with purified recombinant mouse Factor I (1µg/well) in coating buffer. Next day, plates were blocked with 1% BSA in TBS-T for 2 hours at room temperature. During this incubation, dilutions of mouse serum (5% down- wards) or purified mouse Factor I (2µg/ml downwards) were prepared in 1% BSA/150mM NaCl. Next the dilutions were mixed 1:1 with the determined concentrations of purified and biotinylated α-mFI IgGs, i.e. 150µg/ml. The samples were incubated at room temperature for 1 hour on a plate shaker and then loaded onto the coated plate. After one hour incubation, the plate was extensively washed and bound biotinylated α-Factor I antibody was detected with Extravidin-alkaline phosphatase at a concentration of 1:5000. The plates were washed 4x with TBS-T and the assay was developed using Fast p-Nitrophenyl Phosphate Tablets (Sigma) and measured (405nm).

### Western blot

Western blot analysis was performed to detect specific proteins or to confirm their presence in a sample. First, gel electrophoresis was performed to separate the proteins according on their size. The protein samples were boiled up in 4x loading buffer to denature them; β - Mercaptoethanol was added if reduced protein was required for analysis. SDS gel electrophoresis was performed on a 4-12% bis-tris protein gel (Invitrogen) for 50 minutes at 200 V. After separating the proteins, a wet transfer was performed to transfer the separated proteins onto a PVDF membrane where target proteins can detected with specific antibodies.

The blot was assembled by stacking in transfer buffer-equilibrated blotting paper, gel and membrane (first activated with MeOH) into a transfer cassette. The transfer was performed for 1 hour at 350mA, 60W.

Next, the membrane was blocked for 30 minutes on a rotator in blocking buffer. Primary and secondary antibody were both incubated for 1 hour rolling, in between which the membrane was washed 3x 5 minutes in wash buffer. The last washes were done in TBS-T, pH 8, because both used substrates gave stronger signals when the last washes have been in slightly alkaline pH. Depending on the conjugate of the secondary antibody, different substrates were used: proteins were detected chromogenically by addition of 3 ml of TMB or AP substrate (Life Technologies) or, in case of peroxidase conjugated antibodies were also detected chemiluminescently after addition of ECL reagent.

### Preparation of serum

Serum preparation was performed as described in (Lachmann, J. Immunol. Methods, 352(1-2):195-197, January 2010). Blood was taken under sterile conditions and left to clot at room temperature. The initial centrifugation is usually carried out in a bench centrifuge at about 3.000xg for about 5-10 minutes at room temperature. The serum is taken and a second, high speed, centrifugation at; 20.000 xg for 2-5 minutes is carried out. This step is essential to remove all fragments of RBCs that can later distort the results. The serum removed from this second centrifugation can then be chilled, aliquoted and frozen for future experiment. It should be noted that serum used for functional assays should never be stored at temperatures above -80°C. Repeated freeze/thawing should be avoided by preparation of aliquots.

### In vitro assays

Furin digest of pro-Factor I Both, Hek and CHO cells, were secreting only partially processed mFI and the majority was the inactive pro-enzyme of Factor I. In order to get active Factor I, the purified pro-enzyme was digested with the protease furin. In brief, purified mFI was dialysed against TBS and then adjusted to 10mM CaCl2. 1 unit of furin was added per 25µg mFI. The reaction was carried out overnight at 30°C. On the next day, Factor I was used immediately or aliquoted and stored at -80°C.

C3b cleavage assay In a C3b cleavage assay, C3b was first prepared by limited tryptic digest as described in (Bokisch, et al., J. Exp. Med., 129(5):1109-1130, May 1969). In brief, 2µl human C3 were incubated with 0.8µl trypsin (10µg/ml) for exactly 60 seconds at 20°C. The reaction was stopped by addition of 2.4 µl soy-bean trypsin inhibitor (10 µg/ml). This limited digest results in generation of C3b of C3 by cleaving off C3a. Next, C3b was mixed with various amounts of murine Factor I or human Factor I and 2 µl human Factor H and the reaction volume was completed to 10µl with TBS. Alternatively, murine serum was added as a source of Factor I. The reaction was carried out for 30-60 minutes at 37°C and then stopped by addition of reducing 4x loading buffer and boiling. The samples were either analysed by western blot or stored at 4°C.

Time course In order to test the ability of recombinant mouse Factor I to cleave C3b and iC3b, a time course assay was developed. For this, serum was thawed rapidly at 37°C, vortexed briefly, and then placed on ice. The serum was then mixed with recombinant human or mouse Factor I, and then zymosan was added to a final concentration of 5%. Alternative pathway buffer was used as buffer in this assay. Once prepared, the mixture was rotated in a 37°C incubator and at selected time points 40µl of each sample were removed into 10µl of 50mM EDTA. The sampling times were: 0, 30, 60, 120, 180, 240, 480, 600 and 1440 minutes. At each time point the samples were microfuged to remove the zymosan and frozen at -80°C until tested by ELISA. Alternatively, the assay was also done with LPS as complement activating reagent which was the advantage that it is soluble.

The references discussed herein are provided solely for their disclosure prior to the filing date of the present application. Nothing herein is to be construed as an admission that any reference affects the novelty or non-obviousness of the embodiments described herein.

### Sequence Listing

SEQ ID NO: 1
   Coding region of CDS for *Homo sapiens* complement factor I (CFI), transcript variant 2, mRNA, from NCBI Reference Sequence: NM_000204.4: 
atgaagcttc ttcatgtttt cctgttattt ctgtgcttcc acttaaggtt ttgcaaggtc 60
acttatacat ctcaagagga tctggtggag aaaaagtgct tagcaaaaaa atatactcac 120
ctctcctgcg ataaagtctt ctgccagcca tggcagagat gcattgaggg cacctgtgtt 180
tgtaaactac cgtatcagtg cccaaagaat ggcactgcag tgtgtgcaac taacaggaga 240
agcttcccaa catactgtca acaaaagagt ttggaatgtc ttcatccagg gacaaagttt 300
ttaaataacg gaacatgcac agccgaagga aagtttagtg tttccttgaa gcatggaaat 360
acagattcag agggaatagt tgaagtaaaa cttgtggacc aagataagac aatgttcata 420
tgcaaaagca gctggagcat gagggaagcc aacgtggcct gccttgacct tgggtttcaa 480
caaggtgctg atactcaaag aaggtttaag ttgtctgatc tctctataaa ttccactgaa 540
tgtctacatg tgcattgccg aggattagag accagtttgg ctgaatgtac ttttactaag 600
agaagaacta tgggttacca ggatttcgct gatgtggttt gttatacaca gaaagcagat 660
tctccaatgg atgacttctt tcagtgtgtg aatgggaaat acatttctca gatgaaagcc 720
tgtgatggta tcaatgattg tggagaccaa agtgatgaac tgtgttgtaa agcatgccaa 780
ggcaaaggct tccattgcaa atcgggtgtt tgcattccaa gccagtatca atgcaatggt 840
gaggtggact gcattacagg ggaagatgaa gttggctgtg caggctttgc atctgtggct 900
caagaagaaa cagaaatttt gactgctgac atggatgcag aaagaagacg gataaaatca 960
ttattaccta aactatcttg tggagttaaa aacagaatgc acattcgaag gaaacgaatt 1020
gtgggaggaa agcgagcaca actgggagac ctcccatggc aggtggcaat taaggatgcc 1080
agtggaatca cctgtggggg aatttatatt ggtggctgtt ggattctgac tgctgcacat 1140
tgtctcagag ccagtaaaac tcatcgttac caaatatgga caacagtagt agactggata 1200
caccccgacc ttaaacgtat agtaattgaa tacgtggata gaattatttt ccatgaaaac 1260
tacaatgcag gcacttacca aaatgacatc gctttgattg aaatgaaaaa agacggaaac 1320
aaaaaagatt gtgagctgcc tcgttccatc cctgcctgtg tcccctggtc tccttaccta 1380
ttccaaccta atgatacatg catcgtttct ggctggggac gagaaaaaga taacgaaaga 1440
gtcttttcac ttcagtgggg tgaagttaaa ctaataagca actgctctaa gttttacgga 1500
aatcgtttct atgaaaaaga aatggaatgt gcaggtacat atgatggttc catcgatgcc 1560
tgtaaagggg actctggagg ccccttagtc tgtatggatg ccaacaatgt gacttatgtc 1620
tggggtgttg tgagttgggg ggaaaactgt ggaaaaccag agttcccagg tgtttacacc 1680
aaagtggcca attattttga ctggattagc taccatgtag gaaggccttt tatttctcag 1740
tacaatgtat aa 1752
SEQ ID NO: 2
   Translation of the coding region of the CDS for *Homo sapiens* complement factor I (CFI), transcript variant 2, mRNA, from NCBI Reference Sequence: NM_000204.4: 
MKLLHVFLLF LCFHLRFCKV TYTSQEDLVE KKCLAKKYTH LSCDKVFCQP WQRCIEGTCV 60
CKLPYQCPKN GTAVCATNRR SFPTYCQQKS LECLHPGTKF LNNGTCTAEG KFSVSLKHGN 120
TDSEGIVEVK LVDQDKTMFI CKSSWSMREA NVACLDLGFQ QGADTQRRFK LSDLSINSTE 180
CLHVHCRGLE TSLAECTFTK RRTMGYQDFA DVVCYTQKAD SPMDDFFQCV NGKYISQMKA 240
CDGINDCGDQ SDELCCKACQ GKGFHCKSGV CIPSQYQCNG EVDCITGEDE VGCAGFASVA 300
QEETEILTAD MDAERRRIKS LLPKLSCGVK NRMHIRRKRI VGGKRAQLGD LPWQVAIKDA 360
SGITCGGIYI GGCWILTAAH CLRASKTHRY QIWTTVVDWI HPDLKRIVIE YVDRIIFHEN 420
YNAGTYQNDI ALIEMKKDGN KKDCELPRSI PACVPWSPYL FQPNDTCIVS GWGREKDNER 480
VFSLQWGEVK LISNCSKFYG NRFYEKEMEC AGTYDGSIDA CKGDSGGPLV CMDANNVTYV 540
WGVVSWGENC GKPEFPGVYT KVANYFDWIS YHVGRPFISQ YNV 583
SEQ ID NO: 3
   Coding region of CDS for *Homo sapiens* complement factor I (CFI), transcript variant 2, mRNA, from NCBI Reference Sequence: NM_000204.4, without the sequence encoding the N-terminal 18 residue signal sequence: 
aaggtcactt atacatctca agaggatctg gtggagaaaa agtgcttagc aaaaaaatat 60
actcacctct cctgcgataa agtcttctgc cagccatggc agagatgcat tgagggcacc 120
tgtgtttgta aactaccgta tcagtgccca aagaatggca ctgcagtgtg tgcaactaac 180
aggagaagct tcccaacata ctgtcaacaa aagagtttgg aatgtcttca tccagggaca 240
aagtttttaa ataacggaac atgcacagcc gaaggaaagt ttagtgtttc cttgaagcat 300
ggaaatacag attcagaggg aatagttgaa gtaaaacttg tggaccaaga taagacaatg 360
ttcatatgca aaagcagctg gagcatgagg gaagccaacg tggcctgcct tgaccttggg 420
tttcaacaag gtgctgatac tcaaagaagg tttaagttgt ctgatctctc tataaattcc 480
actgaatgtc tacatgtgca ttgccgagga ttagagacca gtttggctga atgtactttt 540
actaagagaa gaactatggg ttaccaggat ttcgctgatg tggtttgtta tacacagaaa 600
gcagattctc caatggatga cttctttcag tgtgtgaatg ggaaatacat ttctcagatg 660
aaagcctgtg atggtatcaa tgattgtgga gaccaaagtg atgaactgtg ttgtaaagca 720
tgccaaggca aaggcttcca ttgcaaatcg ggtgtttgca ttccaagcca gtatcaatgc 780
aatggtgagg tggactgcat tacaggggaa gatgaagttg gctgtgcagg ctttgcatct 840
gtggctcaag aagaaacaga aattttgact gctgacatgg atgcagaaag aagacggata 900
aaatcattat tacctaaact atcttgtgga gttaaaaaca gaatgcacat tcgaaggaaa 960
cgaattgtgg gaggaaagcg agcacaactg ggagacctcc catggcaggt ggcaattaag 1020
gatgccagtg gaatcacctg tgggggaatt tatattggtg gctgttggat tctgactgct 1080
gcacattgtc tcagagccag taaaactcat cgttaccaaa tatggacaac agtagtagac 1140
tggatacacc ccgaccttaa acgtatagta attgaatacg tggatagaat tattttccat 1200
gaaaactaca atgcaggcac ttaccaaaat gacatcgctt tgattgaaat gaaaaaagac 1260
ggaaacaaaa aagattgtga gctgcctcgt tccatccctg cctgtgtccc ctggtctcct 1320
tacctattcc aacctaatga tacatgcatc gtttctggct ggggacgaga aaaagataac 1380
gaaagagtct tttcacttca gtggggtgaa gttaaactaa taagcaactg ctctaagttt 1440
tacggaaatc gtttctatga aaaagaaatg gaatgtgcag gtacatatga tggttccatc 1500
gatgcctgta aaggggactc tggaggcccc ttagtctgta tggatgccaa caatgtgact 1560
tatgtctggg gtgttgtgag ttggggggaa aactgtggaa aaccagagtt cccaggtgtt 1620
tacaccaaag tggccaatta ttttgactgg attagctacc atgtaggaag gccttttatt 1680
tctcagtaca atgtataa 1698
SEQ ID NO: 4
   Translation of the coding region of the CDS for *Homo sapiens* complement factor I (CFI), transcript variant 2, mRNA, from NCBI Reference Sequence: NM_000204.4, without the N-terminal 18 residue signal sequence: 
KVTYTSQEDL VEKKCLAKKY THLSCDKVFC QPWQRCIEGT CVCKLPYQCP KNGTAVCATN 60
RRSFPTYCQQ KSLECLRPGT KFLNNGTCTA EGKFSVSLKH GNTDSEGIVE VKLVDQDKTM 120
FICKSSWSMR EANVACLDLG FQQGADTQRR FKLSDLSINS TECLHVRCRG LETSLAECTF 180
TKRRTMGYQD FADVVCYTQK ADSPMDDFFQ CVNGKYISQM KACDGINDCG DQSDELCCKA 240
CQGKGFHCKS GVCIPSQYQC NGEVDCITGE DEVGCAGFAS VAQEETEILT ADMDAERRRI 300
KSLLPKLSCG VKNRMHIRRK RIVGGKRAQL GDLPWQVAIK DASGITCGGI YIGGCWILTA 360
AHCLRASKTH RYQIWTTVVD WIHPDLKRIV IEYVDRIIFH ENYNAGTYQN DIALIEMKKD 420
GNKKDCELPR SIPACVPWSP YLFQPNDTCI VSGWGREKDN ERVFSLQWGE VKLISNCSKF 480
YGNRFYEKEM ECAGTYDGSI DACKGDSGGP LVCMDANNVT YVWGVVSWGE NCGKPEFPGV 540
YTKVANYFDW ISYHVGRPFI SQYNV 565
SEQ ID NO: 5
   Primer sequence: FI-AAV-F:
   tctagaggat ccgccaccat gaagctc 27
**SEQ ID NO: 6**
   Primer sequence FI-AAV-R:
   aagcttggcg gccgctcaga cattgtgttg agaaacaaga gaccttc 47
SEQ ID NO: 7
   Nucleotide sequence encoding *Homo sapiens* complement factor I (CFI), codon optimized for expression in human cells: 
ATGAAGCTGCTGCATGTCTTTCTGCTGTTTCTGTGCTTCCATCTGCGGTTCTGTAAAGTGACCTATACTAGCCA
GGAGGATCTGGTGGAGAAGAAGTGTCTGGCCAAGAAGTACACACACCTGAGCTGCGACAAGGTGTTCTGTCAGC
CTTGGCAGCGGTGCATCGAGGGCACCTGCGTGTGCAAGCTGCCTTACCAGTGCCCAAAGAACGGCACCGCCGTG
TGCGCCACAAATCGGAGATCTTTTCCAACATATTGCCAGCAGAAGAGCCTGGAGTGTCTGCACCCCGGCACCAA
GTTCCTGAACAATGGCACCTGCACAGCCGAGGGCAAGTTTTCTGTGAGCCTGAAGCACGGCAACACAGATAGCG
AGGGCATCGTGGAGGTGAAGCTGGTGGACCAGGATAAGACCATGTTCATCTGTAAGAGCTCCTGGTCCATGAGG
GAGGCAAACGTGGCATGCCTGGATCTGGGATTCCAGCAGGGAGCAGACACACAGAGGCGCTTTAAGCTGTCCGA
CCTGTCTATCAATAGCACCGAGTGCCTGCACGTGCACTGTAGGGGCCTGGAGACATCCCTGGCAGAGTGCACCT
TCACAAAGCGGAGAACCATGGGCTACCAGGACTTTGCCGACGTGGTGTGCTATACCCAGAAGGCCGATAGCCCC
ATGGACGATTTCTTTCAGTGCGTGAACGGCAAGTATATCTCCCAGATGAAGGCCTGCGACGGCATCAATGACTG
TGGCGATCAGTCTGACGAGCTGTGCTGTAAGGCCTGTCAGGGCAAGGGCTTCCACTGCAAGAGCGGCGTGTGCA
TCCCTTCCCAGTACCAGTGCAACGGCGAGGTGGATTGTATCACAGGAGAGGACGAAGTGGGATGCGCAGGATTT
GCATCTGTGGCACAGGAGGAGACAGAGATCCTGACAGCCGACATGGATGCCGAGAGGCGCCGGATCAAGTCTCT
GCTGCCTAAGCTGAGCTGTGGCGTGAAGAATCGGATGCACATCAGAAGGAAGCGCATCGTGGGAGGCAAGAGGG
CACAGCTGGGCGATCTGCCATGGCAGGTGGCCATCAAGGACGCCTCTGGCATCACCTGCGGCGGCATCTACATC
GGAGGATGTTGGATCCTGACCGCAGCACACTGCCTGAGAGCAAGCAAGACACACAGGTATCAGATCTGGACCAC
AGTGGTGGATTGGATCCACCCAGACCTGAAGAGAATCGTGATCGAGTACGTGGATAGGATCATCTTTCACGAGA
ACTACAATGCCGGCACATATCAGAACGACATCGCCCTGATCGAGATGAAGAAGGATGGCAATAAGAAGGACTGT
GAGCTGCCCAGATCCATCCCTGCATGCGTGCCATGGAGCCCCTATCTGTTCCAGCCCAACGATACCTGCATCGT
GTCCGGATGGGGAAGGGAGAAGGACAATGAGCGGGTGTTTTCTCTGCAGTGGGGCGAGGTGAAGCTGATCTCCA
ACTGTTCTAAGTTCTACGGCAATAGGTTTTATGAGAAGGAGATGGAGTGCGCCGGCACCTACGATGGCAGCATC
GACGCCTGTAAGGGCGATTCCGGAGGACCACTGGTGTGCATGGACGCAAACAATGTGACATACGTGTGGGGAGT
GGTGTCCTGGGGAGAGAACTGCGGCAAGCCAGAGTTCCCCGGCGTATATACCAAGGTGGCCAATTATTTTGATT
GGATTTCCTACCACGTCGGCAGGCCCTTTATTTCCCAGTATAATGTCTAA
SEQ ID NO: 8
   Nucleotide sequence of an HLP liver-specific promoter: 
TGTTTGCTGC TTGCAATGTT TGCCCATTTT AGGGTGGACA CAGGACGCTG TGGTTTCTGA 60
GCCAGGGGGC GACTCAGATC CCAGCCAGTG GACTTAGCCC CTGTTTGCTC CTCCGATAAC 120
TGGGGTGACC TTGGTTAATA TTCACCAGCA GCCTCCCCCG TTGCCCCTCT GGATCCACTG 180
CTTAAATACG GACGAGGACA GGGCCCTGTC TCCTCAGCTT CAGGCACCAC CACTGACCTG 240
GGACAGTGAA T 251
SEQ ID NO: 9
   Nucleotide sequence of an LP1 liver-specific promoter: 
CCCTAAAATG GGCAAACATT GCAAGCAGCA AACAGCAAAC ACACAGCCCT CCCTGCCTGC
TGACCTTGGA GCTGGGGCAG AGGTCAGAGA CCTCTCTGGG CCCATGCCAC CTCCAACATC
CACTCGACCC CTTGGAATTT CGGTGGAGAG GAGCAGAGGT TGTCCTGGCG TGGTTTAGGT
AGTGTGAGAG GGGAATGACT CCTTTCGGTA AGTGCAGTGG AAGCTGTACA CTGCCCAGGC
AAAGCGTCCG GGCAGCGTAG GCGGGCGACT CACATCCCAG CCAGTGGACT TAGCCCCTGT
TTGCTCCTCC GATAACTGGG GTGACCTTGG TTAATATTCA CCAGCAGCCT CCCCCGTTGC
CCCTCTGGAT CCACTGCTTA AATACGGACG AGGACAGGGC CCTGTCTCCT CAGCTTCAGG
CACCACCACT GACCTGGGAC AGTGAAT
SEQ ID NO: 10
   Nucleotide sequence of an HLP2 liver-specific promoter: 
CCCTAAAATG GGCAAACATT GCAAGCAGCA AACAGCAAAC ACACAGCCCT CCCTGCCTGC 60
TGACCTTGGA GCTGGGGCAG AGGTCAGACA CCTCTCTGGG CCCATGCCAC CTCCAACTGG 120
ACACAGGACG CTGTGGTTTC TGAGCCAGGG GGCGACTCAG ATCCCAGCCA GTGGACTTAG 180
CCCCTGTTTG CTCCTCCGAT AACTGGGGTG ACCTTGGTTA ATATTCACCA GCAGCCTCCC 240
CCGTTGCCCC TCTGGATCCA CTGCTTAAAT ACGGACGAGG ACAGGGCCCT GTCTCCTCAG 300
CTTCAGGCAC CACCACTGAC CTGGGACAGT GAATGATCCC CCTGATCTGC GGCC 354
SEQ ID NO: 11
   Amino acid sequence of a MutC capsid protein: 
Met Ala Ala Asp Gly Tyr Leu Pro Asp Trp Leu Glu Asp Asn Leu Ser
1               5                   10                  15
 
Glu Gly Ile Arg Glu Trp Trp Ala Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Gln Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Asp Gln Ser Pro Gln Glu Pro Asp Ser Ser Ser Gly Val Gly
145                 150                 155                 160

Lys Ser Gly Lys Gln Pro Ala Arg Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Ala Pro Thr Ser Leu Gly Ser Asn Thr Met Ala Ser Gly Gly Gly
        195                 200                 205

Ala Pro Met Ala Asp Asn Asn Glu Gly Ala Asp Gly Val Gly Asn Ser
    210                 215                 220
 
Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Lys Leu Ser Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Gln Phe Ser Tyr Thr Phe Glu
                405                 410                 415

Asp Val Pro Phe His Ser Ser Tyr Ala His Ser Gln Ser Leu Asp Arg
            420                 425                 430
 
Leu Met Asn Pro Leu Ile Asp Gln Tyr Leu Tyr Tyr Leu Asn Arg Thr
        435                 440                 445

Gln Gly Thr Thr Ser Gly Thr Thr Asn Gln Ser Arg Leu Leu Phe Ser
    450                 455                 460

Gln Ala Gly Pro Gln Ser Met Ser Leu Gln Ala Arg Asn Trp Leu Pro
465                 470                 475                 480

Gly Pro Cys Tyr Arg Gln Gln Arg Leu Ser Lys Thr Ala Asn Asp Asn
                485                 490                 495

Asn Asn Ser Asn Phe Pro Trp Thr Ala Ala Ser Lys Tyr His Leu Asn
            500                 505                 510

Gly Arg Asp Ser Leu Val Asn Pro Gly Pro Ala Met Ala Ser His Lys
        515                 520                 525

Asp Asp Glu Glu Lys Phe Phe Pro Met His Gly Asn Leu Ile Phe Gly
    530                 535                 540

Lys Glu Gly Thr Thr Ala Ser Asn Ala Glu Leu Asp Asn Val Met Ile
545                 550                 555                 560

Thr Asp Glu Glu Glu Ile Arg Thr Thr Asn Pro Val Ala Thr Glu Gln
                565                 570                 575

Tyr Gly Thr Val Ala Asn Asn Leu Gln Ser Ser Asn Thr Ala Pro Thr
            580                 585                 590

Thr Arg Thr Val Asn Asp Gln Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640
 
Lys His Pro Pro Pro Gln Ile Met Ile Lys Asn Thr Pro Val Pro Ala
                645                 650                 655

Asn Pro Pro Thr Thr Phe Ser Pro Ala Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Asn Lys Ser Val Asn Val Asp Phe Thr Val Asp Thr Asn Gly Val
705                 710                 715                 720

Tyr Ser Glu Pro Arg Pro Ile Gly Thr Arg Tyr Leu Thr Arg Asn Leu
                725                 730                 735
SEQ ID NO: 12
   Amino acid sequence of an LK03 capsid protein:

Met Ala Ala Asp Gly Tyr Leu Pro Asp Trp Leu Glu Asp Asn Leu Ser
1               5                   10                  15

Glu Gly Ile Arg Glu Trp Trp Ala Leu Gln Pro Gly Ala Pro Lys Pro
            20                  25                  30

Lys Ala Asn Gln Gln His Gln Asp Asn Ala Arg Gly Leu Val Leu Pro
        35                  40                  45

Gly Tyr Lys Tyr Leu Gly Pro Gly Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Asp Gln Ser Pro Gln Glu Pro Asp Ser Ser Ser Gly Val Gly
145                 150                 155                 160

Lys Ser Gly Lys Gln Pro Ala Arg Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Ala Pro Thr Ser Leu Gly Ser Asn Thr Met Ala Ser Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Gln Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Lys Leu Ser Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Gln Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Asn Arg Thr
        435                 440                 445

Gln Gly Thr Thr Ser Gly Thr Thr Asn Gln Ser Arg Leu Leu Phe Ser
    450                 455                 460

Gln Ala Gly Pro Gln Ser Met Ser Leu Gln Ala Arg Asn Trp Leu Pro
465                 470                 475                 480

Gly Pro Cys Tyr Arg Gln Gln Arg Leu Ser Lys Thr Ala Asn Asp Asn
                485                 490                 495

Asn Asn Ser Asn Phe Pro Trp Thr Ala Ala Ser Lys Tyr His Leu Asn
            500                 505                 510

Gly Arg Asp Ser Leu Val Asn Pro Gly Pro Ala Met Ala Ser His Lys
        515                 520                 525

Asp Asp Glu Glu Lys Phe Phe Pro Met His Gly Asn Leu Ile Phe Gly
    530                 535                 540

Lys Glu Gly Thr Thr Ala Ser Asn Ala Glu Leu Asp Asn Val Met Ile
545                 550                 555                 560

Thr Asp Glu Glu Glu Ile Arg Thr Thr Asn Pro Val Ala Thr Glu Gln
                565                 570                 575

Tyr Gly Thr Val Ala Asn Asn Leu Gln Ser Ser Asn Thr Ala Pro Thr
            580                 585                 590

Thr Arg Thr Val Asn Asp Gln Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys His Pro Pro Pro Gln Ile Met Ile Lys Asn Thr Pro Val Pro Ala
                645                 650                 655

Asn Pro Pro Thr Thr Phe Ser Pro Ala Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Ile Gln Tyr Thr Ser Asn
    690                 695                 700

Tyr Asn Lys Ser Val Asn Val Asp Phe Thr Val Asp Thr Asn Gly Val
705                 710                 715                 720

Tyr Ser Glu Pro Arg Pro Ile Gly Thr Arg Tyr Leu Thr Arg Pro Leu

725                    730                    735

## Claims

1. A recombinant adeno-associated virus (rAAV) particle for use in preventing, treating, or ameliorating a complement-mediated disorder in a subject in need thereof, wherein the rAAV particle encapsidates a rAAV vector, wherein the rAAV vector comprises a nucleic acid encoding Factor I, or a fragment or derivative thereof that retains C3b-inactivating and iC3b-degradation activity, such that a therapeutically effective amount of the encoded Factor I, or the fragment or derivative thereof, is expressed from the nucleic acid in the subject, thereby increasing the level of C3b-inactivating and iC3b-degradation activity in the subject, wherein the rAAV particle is administered intravenously to the subject,
wherein the encoded Factor I, or the fragment or derivative thereof, is expressed from a liver-specific promoter,
wherein the rAAV particle is pseudotyped to confer liver tropism,
wherein the derivative of Factor I is a polypeptide that retains C3b-inactivating and iC3b-degradation activity and has at least 70% amino acid sequence identity across its entire length to human Factor I with an amino acid sequence of SEQ ID NO: 2 or 4, and
wherein the complement-mediated disorder is age-related macular degeneration (AMD), dense deposit disease (DDD), atypical haemolytic uraemic syndrome (aHUS), C3 glomerulopathies, membranoproliferative glomerulonephritis Type 2 (MPGN2), atherosclerosis, chronic cardiovascular disease, Alzheimer's disease, systemic vasculitis, paroxysmal nocturnal haemoglobinuria (PNH), inflammatory or autoinflammatory diseases of old age, membranoproliferative glomerulonephritis Type 1 (MPGN1), membranoproliferative glomerulonephritis Type III (MPGN3), Guillain-Barré syndrome, Henoch-Schonlein purpura, IgA nephropathy, or membranous glomerulonephritis.

2. A rAAV particle for use according to claim 1, wherein the level of C3b-inactivating and iC3b-degradation activity in the subject is increased to a level that exceeds a normal level, wherein the normal level of C3b-inactivating and iC3b-degradation activity in the subject is equivalent to that provided by 30-40µg/ml Factor I in serum of the subject, preferably wherein the level of C3b-inactivating and iC3b-degradation activity in the subject is increased to:
(i) a level that is up to twice the normal level;
(ii) a level that is up to 80%, or up to 60%, above the normal level;
(iii) a level that is up to 40%, or up to 20% above the normal level; or
(iv) a level that is at least 5%, 10%, 15%, 20%, or 25% above the normal level.

3. A rAAV particle for use according to claim 1 or 2, wherein the rAAV particle infects the liver of the subject following administration, resulting in expression of the Factor I, or the fragment or derivative thereof, from the liver.

4. A rAAV particle for use according to any preceding claim, wherein the rAAV particle comprises one or two AAV2 ITRs and wherein the rAAV particle is pseudotyped with AAV8 capsid protein (rAAV2/8), or pseudotyped with AAV9 capsid protein (rAAV2/9).

5. A rAAV particle for use according to any of claims 1 to 3, wherein the rAAV particle comprises a capsid protein that has an amino acid sequence that has at least 98% or at least 99% amino acid identity along its entire length to the amino acid sequence of SEQ ID NO: 11.

6. A rAAV particle for use according to any preceding claim, wherein the liver-specific promoter is a human alpha-1-anti-trypsin (hAAT) promoter.

7. A rAAV particle for use according to any one of claims 1 to 5, wherein the liver-specific promoter is HLP, LP1, HLP2, hAAT, HCR-hAAT, ApoE-hAAT or LSP.

8. A rAAV particle for use according to any of claims 1 to 5 or 7, wherein the liver-specific promoter comprises a nucleotide sequence that is at least 90% or at least 95% identical over its entire length to the nucleotide sequence of SEQ ID NO: 10.

9. A rAAV particle for use according to any preceding claim, wherein the level of C3b-inactivating and iC3b-degradation activity in the subject is the level in serum of the subject.

10. A rAAV particle for use according to any preceding claim, wherein the complement-mediated disorder is age-related macular degeneration (AMD), preferably early (dry) AMD or geographic atrophy.

11. A rAAV particle for use according to claim 10, wherein the subject is at risk of developing AMD, preferably wherein the subject is homozygous or heterozygous susceptible for one or more SNPs associated with AMD.

12. A rAAV particle for use according to claim 11, wherein the use further comprises determining whether the subject is at risk of developing AMD, preferably wherein it is determined whether the subject is at risk of developing AMD by determining whether the subject is homozygous or heterozygous susceptible for one or more SNPs associated with AMD.

13. A rAAV particle for use according to any of claims 1 to 9, wherein the complement-mediated disorder is dense deposit disease (DDD), atypical haemolytic uraemic syndrome (aHUS), C3 glomerulopathies, membranoproliferative glomerulonephritis Type 2 (MPGN2), systemic vasculitis, paroxysmal nocturnal haemoglobinuria (PNH), membranoproliferative glomerulonephritis type I (MPGN type I), membranoproliferative glomerulonephritis type III (MPGN type III), Henoch-Schonlein purpura or IgA nephropathy.

14. A rAAV particle for use according to any of claims 1 to 9, wherein the complement-mediated disorder is dense deposit disease (DDD), C3 glomerulopathies, membranoproliferative glomerulonephritis Type 2 (MPGN2), paroxysmal nocturnal haemoglobinuria (PNH), membranoproliferative glomerulonephritis type I (MPGN type I), membranoproliferative glomerulonephritis type III (MPGN type III), IgA nephropathy or membranous glomerulonephritis.

15. A rAAV particle for use according to claim 11 or 12, wherein the level of C3b-inactivating and iC3b-degradation activity in the subject is increased to:
(i) a level that is at least 10% above the normal level if the subject is heterozygous susceptible for one or more SNPs associated with AMD; or
(ii) a level that is at least 50% above the normal level if the subject is homozygous susceptible for one or more SNPs associated with AMD;
wherein the normal level of C3b-inactivating and iC3b-degradation activity in the subject is equivalent to that provided by 30-40µg/ml Factor I in serum of the subject.

16. A rAAV particle for use according to any preceding claim, which further comprises determining the level of C3b-inactivating and iC3b-degradation activity in the subject at least a week after the administration, and repeating the administration if the level of activity is found to be at, or below the normal level, wherein the normal level of C3b-inactivating and iC3b-degradation activity in the subject is equivalent to that provided by 30-40µg/ml Factor I in serum of the subject.

17. A rAAV particle for use according to any preceding claim, wherein the fragment or derivative of Factor I is a polypeptide that has at least 95% sequence identity across its entire length to human Factor I with an amino acid sequence of SEQ ID NO: 2 or 4.

18. A rAAV particle for use according to any preceding claim, wherein the fragment or derivative of Factor I is a polypeptide that has at least 99% sequence identity across its entire length to human Factor I with an amino acid sequence of SEQ ID NO: 2 or 4.

19. A rAAV particle for use according to any preceding claim, wherein the Factor I is human Factor I with an amino acid sequence of SEQ ID NO: 2 or 4.

20. A rAAV particle for use according to any preceding claim, wherein the subject is a human subject.

21. A recombinant adeno-associated virus (rAAV) vector which comprises a nucleic acid encoding Factor I, or a fragment or derivative thereof that retains C3b-inactivating and iC3b-degradation activity, wherein the nucleic acid encoding Factor I, or the fragment or derivative thereof, is operably linked to a promoter, wherein the promoter is a liver-specific promoter, and wherein the derivative of Factor I is a polypeptide that retains C3b-inactivating and iC3b-degradation activity and has at least 70% amino acid sequence identity across its entire length to human Factor I with an amino acid sequence of SEQ ID NO: 2 or 4.

22. A rAAV vector according to claim 21, wherein the liver-specific promoter is a human alpha-1-anti-trypsin (hAAT) promoter.

23. A rAAV vector according to claim 21, wherein the liver-specific promoter is HLP, LP1, HLP2, hAAT, HCR-hAAT, ApoE-hAAT or LSP.

24. A rAAV vector according to claim 21 or 23, wherein the liver-specific promoter comprises a nucleotide sequence that is at least 90% or at least 95% identical over its entire length to the nucleotide sequence of SEQ ID NO: 10.

25. A rAAV vector according to any of claims 21-24, which comprises an expression cassette flanked by AAV inverted terminal repeats (ITRs), wherein the expression cassette comprises the nucleic acid encoding Factor I, or the fragment or derivative thereof, operably linked to a promoter and a polyadenylation recognition site, preferably wherein the ITRs are AAV2 ITRs.

26. A rAAV particle, which comprises a rAAV capsid encapsidating a rAAV vector according to any of claims 21-25, wherein the rAAV particle is pseudotyped to confer liver tropism.

27. A rAAV particle according to claim 26, wherein the rAAV particle comprises AAV8 or AAV9 capsid protein, preferably wherein the rAAV particle comprises one or two AAV2 ITRs.

28. A rAAV particle according to claim 26, wherein the rAAV particle comprises a capsid protein that has an amino acid sequence that has at least 98% or at least 99% amino acid identity along its entire length to the amino acid sequence of SEQ ID NO: 11.

29. A rAAV particle according to any one of claims 26-28, wherein the fragment or derivative of Factor I is a polypeptide that has at least 95% sequence identity across its entire length to human Factor I with an amino acid sequence of SEQ ID NO: 2 or 4.

30. A rAAV particle according to any of claims 26-29, wherein the fragment or derivative of Factor I is a polypeptide that has at least 99% sequence identity across its entire length to human Factor I with an amino acid sequence of SEQ ID NO: 2 or 4.

31. A pharmaceutical composition, which comprises: a rAAV vector according to any of claims 21 to 25, or a rAAV particle according to any of claims 26 to 30; and a pharmaceutically acceptable carrier, excipient, or diluent, preferably wherein the pharmaceutical composition is suitable for intravenous administration.

32. A rAAV vector according to any of claims 21 to 25, a rAAV particle according to any of claims 26 to 30, or a pharmaceutical composition according to claim 31, for use as a medicament.

## Patentansprüche

1. Rekombinantes-Adeno-assoziiertes-Virus(rAAV)-Partikel zur Verwendung beim Vorbeugen, Behandeln oder Lindern einer komplementvermittelten Störung bei einem dies benötigenden Individuum, wobei das rAAV-Partikel einen rAAV-Vektor enkapsidiert, wobei der rAAV-Vektor eine Nukleinsäure umfasst, die Faktor I oder ein Fragment oder Derivat davon, bei dem C3b-Inaktivierungs- und iC3b-Abbauaktivität erhalten sind, codiert, so dass eine therapeutisch wirksame Menge des codierten Faktor I oder des Fragments oder Derivats davon von der Nukleinsäure im Individuum exprimiert wird, wodurch der Spiegel von C3b-Inaktivierungs- und iC3b-Abbauaktivität im Individuum erhöht wird, wobei das rAAV-Partikel dem Individuum intravenös verabreicht wird,
wobei der codierte Faktor I oder das Fragment oder Derivat davon von einem leberspezifischen Promotor exprimiert wird,
wobei das rAAV-Partikel pseudotypisiert ist, so dass ein Lebertropismus verliehen wird,
wobei es sich bei dem Derivat von Faktor I um ein Polypeptid handelt, bei dem C3b-Inaktivierungs- und iC3b-Abbauaktivität erhalten sind und das über seine Gesamtlänge eine Aminosäuresequenzidentität von mindestens 70 % mit menschlichem Faktor I mit einer Aminosäuresequenz unter SEQ ID NO: 2 oder 4 aufweist, und wobei es sich bei der komplementvermittelten Störung um altersbedingte Makuladegeneration (AMD), DDD (Dense Deposit Disease), atypisches hämolytisch-urämisches Syndrom (aHUS), C3-Glomerulopathien, membranoproliferative Glomerulonephritis Typ 2 (MPGN2), Atherosklerose, chronische Herz-Kreislauf-Krankheit, Morbus Alzheimer, systemische Vaskulitis, paroxysmale nächtliche Hämoglobinurie (PNH), Entzündungs- oder autoinflammatorische Krankheiten im Alter, membranoproliferative Glomerulonephritis Typ 1 (MPGN1), membranoproliferative Glomerulonephritis Typ III (MPGN3), Guillain-Barré-Syndrom, Purpura Schönlein-Henoch, IgA-Nephropathie oder membranöse Glomerulonephritis handelt.

2. rAAV-Partikel zur Verwendung nach Anspruch 1, wobei der Spiegel von C3b-Inaktivierungs- und iC3b-Abbauaktivität im Individuum auf einen Spiegel erhöht wird, der über einen Normalspiegel hinausgeht, wobei der Normalspiegel von C3b-Inaktivierungs- und iC3b-Abbauaktivität im Individuum dem Spiegel entspricht, der durch 30-40 µg/ml Faktor I im Serum des Individuums bereitgestellt wird, vorzugsweise wobei der Spiegel von C3b-Inaktivierungs- und iC3b-Abbauaktivität im Individuum erhöht wird auf:
(i) einen Spiegel, der bis zu doppelt so hoch ist wie die Normalspiegel;
(ii) einen Spiegel, der bis zu 80 % oder bis zu 60 % über dem Normalspiegel liegt;
(iii) einen Spiegel, der bis zu 40 % oder bis zu 20 % über dem Normalspiegel liegt; oder
(iv) einen Spiegel, der mindestens 5 %, 10 %, 15 %, 20 % oder 25 % über dem Normalspiegel liegt.

3. rAAV-Partikel zur Verwendung nach Anspruch 1 oder 2, wobei das rAAV-Partikel nach Verabreichung die Leber des Individuums infiziert, was zur Expression des Faktor I oder des Fragments oder Derivats davon aus der Leber führt.

4. rAAV-Partikel zur Verwendung nach einem vorhergehenden Anspruch, wobei das rAAV-Partikel ein oder zwei AAV2-ITR umfasst und wobei das rAAV-Partikel mit AAV8-Kapsidprotein pseudotypisiert ist (rAAV2/8) oder mit AAV9-Kapsidprotein pseudotypisiert ist(rAAV2/9).

5. rAAV-Partikel zur Verwendung nach einem der Ansprüche 1 bis 3, wobei das rAAV-Partikel ein Kapsidprotein umfasst, das eine Aminosäuresequenz aufweist, die über ihre gesamte Länge eine Aminosäureidentität von mindestens 98 % oder mindestens 99 % mit der Aminosäuresequenz unter SEQ ID NO: 11 aufweist.

6. rAAV-Partikel zur Verwendung nach einem vorhergehenden Anspruch, wobei es sich bei dem leberspezifischen Promotor um einen hAAT(human Alpha-1-Anti-Trypsin)-Promotor handelt.

7. rAAV-Partikel zur Verwendung nach einem der Ansprüche 1 bis 5, wobei es sich bei dem leberspezifischen Promotor um HLP, LP1, HLP2, hAAT, HCR-hAAT, ApoE-hAAT oder LSP handelt.

8. rAAV-Partikel zur Verwendung nach einem der Ansprüche 1 bis 5 oder 7, wobei der leberspezifische Promotor eine Nukleotidsequenz umfasst, die über ihre gesamte Länge zu mindestens 90 % oder mindestens 95 % mit der Nukleotidsequenz unter SEQ ID NO: 10 identisch ist.

9. rAAV-Partikel zur Verwendung nach einem vorhergehenden Anspruch, wobei es sich bei dem Spiegel von C3b-Inaktivierungs- und iC3b-Abbauaktivität im Individuum um den Serumspiegel im Individuum handelt.

10. rAAV-Partikel zur Verwendung nach einem vorhergehenden Anspruch, wobei es sich bei der komplementvermittelten Störung um altersabhängige Makuladegeneration (AMD), vorzugsweise frühe (trockene) AMD oder geografische Atrophie handelt.

11. rAAV-Partikel zur Verwendung nach Anspruch 10, wobei bei dem Individuum ein Risiko für die Entwicklung von AMD besteht, vorzugsweise wobei das Individuum für einen oder mehrere mit AMD assoziierte SNP homozygot oder heterozygot empfänglich ist.

12. rAAV-Partikel zur Verwendung nach Anspruch 11, wobei die Verwendung ferner Bestimmen, ob bei dem Individuum ein Risiko für die Entwicklung von AMD besteht, vorzugsweise wobei durch Bestimmen, ob das Individuum für einen oder mehrere mit AMD assoziierte SNP homozygot oder heterozygot empfänglich ist, bestimmt wird, ob bei dem Individuum ein Risiko für die Entwicklung von AMD besteht.

13. rAAV-Partikel zur Verwendung nach einem der Ansprüche 1 bis 9, wobei es sich bei der komplementvermittelten Störung um DDD (Dense Deposit Disease), atypisches hämolytisch-urämisches Syndrom (aHUS), C3-Glomerulopathien, membranoproliferative Glomerulonephritis Typ 2 (MPGN2), systemische Vaskulitis, paroxysmale nächtliche Hämoglobinurie (PNH), membranoproliferative Glomerulonephritis Typ I (MPGN Typ I), membranoproliferative Glomerulonephritis Typ III (MPGN Typ III), Purpura Schönlein-Henoch oder IgA-Nephropathie handelt.

14. rAAV-Partikel zur Verwendung nach einem der Ansprüche 1 bis 9, wobei es sich bei der komplementvermittelten Störung um DDD (Dense Deposit Disease), C3-Glomerulopathien, membranoproliferative Glomerulonephritis Typ 2 (MPGN2), paroxysmale nächtliche Hämoglobinurie (PNH), membranoproliferative Glomerulonephritis Typ I (MPGN Typ I), membranoproliferative Glomerulonephritis Typ III (MPGN Typ III), IgA-Nephropathie oder membranöse Glomerulonephritis handelt.

15. rAAV-Partikel zur Verwendung nach Anspruch 11 oder 12, wobei der Spiegel von C3b-Inaktivierungs- und iC3b-Abbauaktivität im Individuum erhöht wird auf:
(i) einen Spiegel, der mindestens 10 % über dem Normalspiegel liegt, wenn das Individuum für einen oder mehrere mit AMD assoziierte SNP heterozygot empfänglich ist; oder
(ii) einen Spiegel, der mindestens 50 % über dem Normalspiegel liegt, wenn das Individuum für einen oder mehrere mit AMD assoziierte SNP homozygot empfänglich ist;
wobei der Normalspiegel von C3b-Inaktivierungs- und iC3b-Abbauaktivität im Individuum dem Spiegel entspricht, der durch 30-40 µg/ml Faktor I im Serum des Individuums bereitgestellt wird.

16. rAAV-Partikel zur Verwendung nach einem vorhergehenden Anspruch, die ferner Bestimmen des Spiegels von C3b-Inaktivierungs- und iC3b-Abbauaktivität im Individuum wenigstens eine Woche nach der Verabreichung und Wiederholen der Verabreichung, wenn sich herausstellt, dass der Aktivitätsspiegel auf oder unter dem Normalspiegel liegt, umfasst, wobei der Normalspiegel von C3b-Inaktivierungs- und iC3b-Abbauaktivität im Individuum dem Spiegel entspricht, der durch 30-40 µg/ml Faktor I im Serum des Individuums bereitgestellt wird.

17. rAAV-Partikel zur Verwendung nach einem vorhergehenden Anspruch, wobei es sich bei dem Fragment oder Derivat von Faktor I um ein Polypeptid handelt, das über seine Gesamtlänge eine Sequenzidentität von mindestens 95 % mit menschlichem Faktor I mit einer Aminosäuresequenz unter SEQ ID NO: 2 oder 4 aufweist.

18. rAAV-Partikel zur Verwendung nach einem vorhergehenden Anspruch, wobei es sich bei dem Fragment oder Derivat von Faktor I um ein Polypeptid handelt, das über seine Gesamtlänge eine Sequenzidentität von mindestens 99 % mit menschlichem Faktor I mit einer Aminosäuresequenz unter SEQ ID NO: 2 oder 4 aufweist.

19. rAAV-Partikel zur Verwendung nach einem vorhergehenden Anspruch, wobei es sich bei dem Faktor I um menschlichen Faktor I mit einer Aminosäuresequenz unter SEQ ID-NO: 2 oder 4 handelt.

20. rAAV-Partikel zur Verwendung nach einem vorhergehenden Anspruch, wobei es sich bei dem Individuum um einen Menschen handelt.

21. Rekombinantes-Adeno-assoziiertes-Virus(rAAV)-Vektor, der eine Nukleinsäure umfasst, die Faktor I oder ein Fragment oder Derivat davon, bei dem C3b-Inaktivierungs- und iC3b-Abbauaktivität erhalten sind, codiert, wobei die Faktor I oder das Fragment oder Derivat davon codierende Nukleinsäure in operativer Verknüpfung mit einem Promotor steht, wobei es sich bei dem Promotor um einen leberspezifischen Promotor handelt und wobei es sich bei dem Derivat von Faktor I um ein Polypeptid handelt, bei dem C3b-Inaktivierungs- und iC3b-Abbauaktivität erhalten sind und das über seine Gesamtlänge eine Aminosäuresequenzidentität von mindestens 70 % mit menschlichem Faktor I mit einer Aminosäuresequenz unter SEQ ID NO: 2 oder 4 aufweist.

22. rAAV-Vektor nach Anspruch 21, wobei es sich bei dem leberspezifischen Promotor um einen hAAT(human Alpha-1-Anti-Trypsin)-Promotor handelt.

23. rAAV-Vektor nach Anspruch 21, wobei es sich bei dem leberspezifischen Promotor um HLP, LP1, HLP2, hAAT, HCR-hAAT, ApoE-hAAT oder LSP handelt.

24. rAAV-Vektor nach Anspruch 21 oder 23, wobei der leberspezifische Promotor eine Nukleotidsequenz umfasst, die über ihre gesamte Länge zu mindestens 90 % oder mindestens 95 % mit der Nukleotidsequenz unter SEQ ID NO: 10 identisch ist.

25. rAAV-Vektor nach einem der Ansprüche 21-24, der eine von AAV-ITR (Inverted Terminal Repeats) flankierte Expressionskassette umfasst, wobei die Expressionskassette die Faktor I oder das Fragment oder Derivat davon codierende Nukleinsäure in operativer Verknüpfung mit einem Promotor und einer Polyadenylierungserkennungsstelle umfasst, vorzugsweise wobei es sich bei den ITR um AAV2-ITR handelt.

26. rAAV-Partikel, das ein rAAV-Kapsid umfasst, das einen rAAV-Vektor nach einem der Ansprüche 21-25 enkapsidiert, wobei das rAAV-Partikel pseudotypisiert ist, so dass ein Lebertropismus verliehen wird.

27. rAAV-Partikel nach Anspruch 26, wobei das rAAV-Partikel AAV8- oder AAV9-Kapsidprotein umfasst, vorzugsweise wobei das rAAV-Partikel ein oder zwei AAV2-ITR umfasst.

28. rAAV-Partikel nach Anspruch 26, wobei das rAAV-Partikel ein Kapsidprotein umfasst, das eine Aminosäuresequenz aufweist, die über ihre gesamte Länge eine Aminosäureidentität von mindestens 98 % oder mindestens 99 % mit der Aminosäuresequenz unter SEQ ID NO: 11 aufweist.

29. rAAV-Partikel nach einem der Ansprüche 26-28, wobei es sich bei dem Fragment oder Derivat von Faktor I um ein Polypeptid handelt, das über seine Gesamtlänge eine Sequenzidentität von mindestens 95 % mit menschlichem Faktor I mit einer Aminosäuresequenz unter SEQ ID NO: 2 oder 4 aufweist.

30. rAAV-Partikel nach einem der Ansprüche 26-29, wobei es sich bei dem Fragment oder Derivat von Faktor I um ein Polypeptid handelt, das über seine Gesamtlänge eine Sequenzidentität von mindestens 99 % mit menschlichem Faktor I mit einer Aminosäuresequenz unter SEQ ID NO: 2 oder 4 aufweist.

31. Pharmazeutische Zusammensetzung, die Folgendes umfasst: einen rAAV-Vektor nach einem der Ansprüche 21 bis 25 oder ein rAAV-Partikel nach einem der Ansprüche 26 bis 30; und ein pharmazeutisch unbedenkliches Träger-, Hilfs- oder Verdünnungsmittel, vorzugsweise wobei die pharmazeutische Zusammensetzung zur intravenösen Verabreichung geeignet ist.

32. rAAV-Vektor nach einem der Ansprüche 21 bis 25, rAAV-Partikel nach einem der Ansprüche 26 bis 30 oder pharmazeutische Zusammensetzung nach Anspruch 31 zur Verwendung als Arzneimittel.

## Revendications

1. Particule de virus adéno-associé recombinant (rAAV) pour une utilisation dans la prévention, le traitement ou l'amélioration d'un trouble médié par le complément chez un sujet en ayant besoin, la particule rAAV encapsidant un vecteur rAAV, le vecteur rAAV comprenant un acide nucléique codant pour le facteur I, ou un fragment ou un dérivé de celui-ci, qui conserve une activité d'inactivation de C3b et de dégradation d'iC3b, de telle sorte qu'une quantité thérapeutiquement efficace du facteur I codé, ou du fragment ou dérivé de celui-ci, est exprimée à partir de l'acide nucléique chez le sujet, augmentant ainsi le taux d'activité d'inactivation de C3b et de dégradation d'iC3b, la particule rAAV étant administrée par voie intraveineuse au sujet,
le facteur I codé, ou le fragment ou dérivé de celui-ci, étant exprimé à partir d'un promoteur hépatique spécifique,
La particule rAAV étant pseudotypée pour conférer un tropisme hépatique,
le dérivé du facteur I étant un polypeptide qui conserve l'activité d'inactivation de C3b et de dégradation d'iC3b, et possède une séquence d'acides aminés au moins 70 % identique sur sa longueur totale au facteur I humain ayant une séquence d'acides aminés de SEQ ID NO : 2 ou 4, et
le trouble médié par le complément étant la dégénérescence maculaire liée à l'âge (DMLA), la maladie du dépôt dense (MDD), le syndrome hémolytique et urémique atypique (SHUa), les glomérulopathies à C3, la glomérulonéphrite membranoproliférative de type 2 (MPGN2), l'athérosclérose, les maladies cardiovasculaires chroniques, la maladie d'Alzheimer, la vascularite systémique, l'hémoglobinurie paroxystique nocturne (HPN), les maladies inflammatoires ou auto-inflammatoires liées à l'âge, la glomérulonéphrite membranoproliférative de type 1 (MPGN1), la glomérulonéphrite membranoproliférative de type III (MPGN3), le syndrome de Guillain-Barré, le purpura de Henoch-Schönlein, la néphropathie à IgA ou la glomérulonéphrite membraneuse.

2. Particule rAAV selon la revendication 1, le taux d'activité d'inactivation de C3b et de dégradation d'iC3b chez le sujet étant augmenté à un taux supérieur à la normale, le taux normal d'activité d'inactivation de C3b et de dégradation d'iC3b étant équivalent à celui produit par 30 à 40 µg/ml de facteur I dans le sérum du sujet, de préférence, le taux d'activité d'inactivation de C3b et de dégradation d'iC3b chez le sujet étant augmenté jusqu'à :
(i) un taux qui est jusqu'à deux fois supérieur à la normale ;
(ii) un taux qui est jusqu'à 80 %, ou jusqu'à 60 %, au-dessus de la normale ;
(iii) un taux qui est jusqu'à 40 %, ou jusqu'à 20 %, au-dessus de la normale ; ou
(iv) un taux qui est au moins 5 %, 10 %, 15 %, 20 % ou 25 % au-dessus de la normale.

3. Particule rAAV selon la revendication 1 ou 2, la particule rAAV infectant le foie du sujet après l'administration, entraînant l'expression du facteur I, ou d'un fragment ou dérivé de celui-ci, à partir du foie.

4. Particule rAAV selon l'une quelconque des revendications précédentes, la particule rAAV comprenant une ou deux ITR d'AAV2 et la particule rAAV étant pseudotypée avec la protéine de capside AAV8 (rAAV2/8) ou pseudotypée avec la protéine de capside AAV9 (rAAV2/9).

5. Particule rAAV selon l'une quelconque des revendications 1 à 3, la particule rAAV comprenant une protéine de capside qui a une séquence d'acides aminés qui présente au moins 98 % ou 99 % d'identité sur sa longueur totale avec la séquence d'acides aminés de SEQ ID NO : 11.

6. Particule rAAV selon l'une quelconque des revendications précédentes, le promoteur hépatique spécifique étant un promoteur de l'alpha-1-antitrypsine humaine (hAAT).

7. Particule rAAV selon l'une quelconque des revendications 1 à 5, le promoteur hépatique spécifique étant HLP, LP1, HLP2, hAAT, HCR-hAAT, ApoE-hAAT ou LSP.

8. Particule rAAV selon l'une quelconque des revendications 1 à 5 ou 7, le promoteur hépatique spécifique comprenant une séquence nucléotidique qui est au moins 90 % ou au moins 95 % identique sur sa longueur totale à la séquence nucléotidique de SEQ ID NO : 10.

9. Particule rAAV selon l'une quelconque des revendications précédentes, le taux d'activité d'inactivation de C3b et de dégradation d'iC3b chez le sujet étant le taux sérique du sujet.

10. Particule rAAV selon l'une quelconque des revendications précédentes, le trouble médié par le complément étant la dégénérescence maculaire liée à l'âge (DMLA), de préférence une DMLA précoce (sèche) ou une atrophie géographique.

11. Particule rAAV selon la revendication 10, le sujet présentant un risque de développer une DMLA, de préférence, le sujet étant homozygote ou hétérozygote susceptible d'être porteur d'un ou plusieurs SNP associés à la DMLA.

12. Particule rAAV selon la revendication 11, l'utilisation comprenant en outre la détermination du risque de développement de DMLA chez le sujet, de préférence, il est déterminé si le sujet risque de développer la DMLA en déterminant si le sujet est homozygote ou hétérozygote susceptible d'un ou plusieurs SNP associés à la DMLA.

13. Particule rAAV selon l'une quelconque des revendications 1 à 9, le trouble médié par le complément étant la maladie du dépôt dense (MDD), le syndrome hémolytique et urémique atypique (SHUa), les glomérulopathies C3, la glomérulonéphrite membranoproliférative de type 2 (MPGN2), la vascularite systémique, l'hémoglobinurie paroxystique nocturne (HPN), la glomérulonéphrite membranoproliférative de type I (MPGN de type I), la glomérulonéphrite membranoproliférative de type III (MPGN type III), le purpura de Henoch-Schönlein ou la néphropathie à IgA.

14. Particule rAAV selon l'une quelconque des revendications 1 à 9, le trouble médié par le complément étant la maladie du dépôt dense (MDD), le syndrome hémolytique et urémique atypique (SHUa), les glomérulopathies C3, la glomérulonéphrite membranoproliférative de type 2 (MPGN2), l'hémoglobinurie paroxystique nocturne (HPN), la glomérulonéphrite membranoproliférative de type I (MPGN de type I), la glomérulonéphrite membranoproliférative de type III (MPGN type III), le purpura de Henoch-Schönlein, la néphropathie à IgA ou la glomérulonéphrite membraneuse.

15. Particule rAAV selon la revendication 11 ou 12, le taux d'activité d'inactivation de C3b et de dégradation d'iC3b chez le sujet étant augmenté à :
(i) un taux qui est au moins 10 % au-dessus de la normale si le sujet est hétérozygote susceptible d'un ou plusieurs SNP associés à la DMLA ; ou
(ii) un taux qui est au moins 50 % au-dessus de la normale si le sujet est homozygote susceptible d'un ou plusieurs SNP associés à la DMLA ;
le taux normal d'activité d'inactivation de C3b et de dégradation d'iC3b chez le sujet étant équivalent à celui produit par 30 à 40 µg/ml de facteur I dans le sérum du sujet.

16. Particule rAAV selon l'une quelconque des revendications précédentes, qui comprend en outre la détermination du taux d'activité d'inactivation de C3b et de dégradation d'iC3b chez le sujet au moins une semaine après l'administration, et la répétition de l'administration si le taux d'activité est égal ou inférieur à la normale, le taux normal d'activité d'inactivation de C3b et de dégradation d'iC3b chez le sujet étant équivalent à celui produit par 30 à 40 µg/ml de facteur I dans le sérum du sujet.

17. Particule rAAV selon l'une quelconque des revendications précédentes, le fragment ou dérivé du facteur I étant un polypeptide qui présente au moins 95 % d'identité sur sa longueur totale avec le facteur I humain ayant une séquence d'acides aminés de SEQ ID NO : 2 ou 4.

18. Particule rAAV selon l'une quelconque des revendications précédentes, le fragment ou dérivé du facteur I étant un polypeptide qui présente au moins 99 % d'identité sur sa longueur totale avec le facteur I humain ayant une séquence d'acides aminés de SEQ ID NO : 2 ou 4.

19. Particule rAAV selon l'une quelconque des revendications précédentes, le facteur I étant un facteur I humain ayant une séquence d'acides aminés de SEQ ID NO : 2 ou 4.

20. Particule rAAV selon l'une quelconque des revendications précédentes, le sujet étant un sujet humain.

21. Vecteur de virus adéno-associé recombinant (rAAV) qui comprend un acide nucléique codant pour le facteur I, ou un fragment ou un dérivé de celui-ci, qui conserve une activité d'inactivation de C3b et de dégradation d'iC3b, l'acide nucléique codant pour le facteur I, ou un fragment ou un dérivé de celui-ci, étant lié fonctionnellement à un promoteur, le promoteur étant un promoteur hépatique spécifique, le dérivé du facteur I étant un polypeptide qui conserve l'activité d'inactivation de C3b et de dégradation d'iC3b, et possède une séquence d'acides aminés au moins 70 % identique sur sa longueur totale au facteur I humain ayant une séquence d'acides aminés de SEQ ID NO : 2 ou 4.

22. Vecteur rAAV selon la revendication 21, le promoteur hépatique spécifique étant un promoteur de l'alpha-1-antitrypsine humaine (hAAT).

23. Vecteur rAAV selon la revendication 21, le promoteur hépatique spécifique étant HLP, LP1, HLP2, hAAT, HCR-hAAT, ApoE-hAAT ou LSP.

24. Vecteur rAAV selon la revendication 21 ou 23, le promoteur hépatique spécifique comprenant une séquence nucléotidique qui est au moins 90 % ou au moins 95 % identique sur sa longueur totale à la séquence nucléotidique de SEQ ID NO : 10.

25. Vecteur rAAV selon l'une quelconque des revendications 21 à 24, qui comprend une cassette d'expression flanquée par des répétitions terminales inversées (ITR) d'AAV, la cassette d'expression comprenant l'acide nucléique codant pour le facteur I, ou un fragment ou un dérivé de celui-ci, lié fonctionnellement à un promoteur et à un site de reconnaissance de polyadénylation, de préférence les ITR étant des ITR d'AAV2.

26. Particule rAAV comprenant une capside de rAAV encapsidant un vecteur rAAV selon l'une quelconque des revendications 21 à 25, la particule rAAV étant pseudotypée pour conférer un tropisme hépatique.

27. Particule rAAV selon la revendication 26, comprenant la protéine de capside AAV8 ou AAV9, de préférence la particule rAAV comprenant une ou deux ITR d'AAV2.

28. Particule rAAV selon la revendication 26, la particule rAAV comprenant une protéine de capside qui a une séquence d'acides aminés qui présente au moins 98 % ou 99 % d'identité sur sa longueur totale avec la séquence d'acides aminés de SEQ ID NO : 11.

29. Particule rAAV selon l'une quelconque des revendications 26 à 28, le fragment ou dérivé du facteur I étant un polypeptide qui présente au moins 95 % d'identité sur sa longueur totale avec le facteur I humain avec une séquence d'acides aminés de SEQ ID NO : 2 ou 4.

30. Particule rAAV selon l'une quelconque des revendications 26 à 29, le fragment ou dérivé du facteur I étant un polypeptide qui présente au moins 99 % d'identité sur sa longueur totale avec le facteur I humain avec une séquence d'acides aminés de SEQ ID NO : 2 ou 4.

31. Composition pharmaceutique, qui comprend : un vecteur rAAV selon l'une quelconque des revendications 21 à 25, ou une particule rAAV selon l'une quelconque des revendications 26 à 30 ; et un véhicule, excipient ou diluant pharmaceutiquement acceptable, de préférence, la composition pharmaceutique étant adaptée à une administration intraveineuse.

32. Vecteur rAAV selon l'une quelconque des revendications 21 à 25, particule rAAV selon l'une quelconque des revendications 26 à 30, ou composition pharmaceutique selon la revendication 31, pour une utilisation en tant que médicament.
